# FASCICULE DE BREVET EUROPEEN

(11) **EP 2 334 796 B1**
(45) Date de publication et mention de la délivrance du brevet: **04.03.2015**
(21) Numéro de dépôt: 09740348.9
(22) Date de dépôt: 15.09.2009
(51) Int. Cl.: C12N 15/82, C12N 15/11, C12N 15/29, C12N 15/33, A01H 5/00

(54) **IMPORTATION D'UN RIBOZYME DANS LES MITOCHONDRIES VEGETALES PAR UN PSEUDO-ARNt AMINOACYLABLE PAR LA VALINE**
EINSCHLEUSUNG EINES RIBOZYMS IN PFLANZENMITOCHONDRIEN ÜBER EINE DURCH VALIN AMINOACYLIERBARE PSEUDO-TRNA
IMPORTATION OF A RIBOZYME INTO VEGETABLE MITOCHONDRIA BY A PSEUDO-TRNA THAT CAN BE AMINOACYLATED BY VALINE

(30) Priorité: 16.09.2008 FR 0805060
(43) Date de publication de la demande: 22.06.2011
(73) Titulaire: Centre National de la Recherche Scientifique, 75016 Paris (FR)
(72) Inventeur: DIETRICH, André, F-67203 Oberschaeffolsheim (FR); VAL, Romain, F-78200 Mantes la Jolie (FR); VALENTIN, Clarisse, F-67000 Strasbourg (FR); DREHER, Theo, Corvallis Oregon 97330 (US); BARCISZEWSKI, Jan, PL-60334 Poznan (PL); SZYMANSKI, Maciej, PL-60133 Poznan (PL); WYSZKO, Eliza, PL-61465 Poznan (PL)
(74) Mandataire: Rançon, Xavier Lucien Abel
(86) Numéro de dépôt international: PCT/FR2009/001094
(87) Numéro de publication internationale: WO 2010/031918

(56) Documents cités:
- WO-A-94/18334
- WO-A-96/00232
- VAL ROMAIN ET AL.: "Manipulation de l'expression génétique dans les mitochondries de plante par l'import de séquences passagères associées à une structure de type ARNt" 6ÈME RENCONTR SIFRARN, 2 décembre 2006 (2006-12-02), XP002512808 Extrait de l'Internet: URL:http://sifrarn2006.univ-rennes1.fr/Res umes/Recueil actes complet.pdf> [extrait le 2009-01-29]
- MATSUDA D ET AL: "The tRNA-like structure of Turnip yellow mosaic virus RNA is a 3'-translational enhancer" VIROLOGY, ACADEMIC PRESS,ORLANDO, US, vol. 321, no. 1, 30 mars 2004 (2004-03-30), pages 36-46, XP004497041 ISSN: 0042-6822
- SIQUEIRA S F ET AL: "Marchantia polymorpha mitochondrial orf identifies transcribed sequence in angiosperm mitochondrial genome." BIOCHIMICA ET BIOPHYSICA ACTA 21 SEP 2001, vol. 1520, no. 3, 21 septembre 2001 (2001-09-21), pages 203-211, XP002512809 ISSN: 0006-3002
- DATABASE EMBL [Online] 16 juin 2003 (2003-06-16), "Arabidopsis thaliana clone D10 mRNA sequence." XP002512811 extrait de EBI accession no. EMBL:AY299283 Database accession no. AY299283
- BUSSIÈRE FRÉDÉRIC ET AL: "Development of an efficient cis-trans-cis ribozyme cassette to inactivate plant genes." PLANT BIOTECHNOLOGY JOURNAL NOV 2003, vol. 1, no. 6, novembre 2003 (2003-11), pages 423-435, XP002512810 ISSN: 1467-7652

### SEQUENCE LISTING

<110> CENTRE NATIONAL DE LA RECHERCHE SCIENTIFIQUE
   DIETRICH, André
   VAL, Romain
   VALENTIN, Clarisse
   DREHER, Theo
   BARCISZEWSKI, Jan
   SZYMANSKI, Maciej
   WYSZKO, Eliza
<120> Importation d'un ribozyme dans les mitochondries végétales par un pseudo-ARNt aminoacylable par la valine
<130> F644/204/Ext
<160> 66
<170> PatentIn version 3.3
<210> 1
   <211> 278
   <212> DNA
   <213> Artificial
<220>
   <223> Séquence complète de la construction de gène utilisée pour exprimer et importer un trans-ribozyme dirigé contre l'ARNm atp9 dans les mitochondries in vivo
<220>
   <221> misc_feature
   <222> (1)..(33)
   <223> séquence exprimant le trans-ribozyme dirigé contre l'ARNm atp9 (tRzatp9)
<220>
   <221> misc_feature
   <222> (34)..(75)
   <223> séquence exprimant la séquence de liaison (L)
<220>
   <221> misc_feature
   <222> (76)..(195)
   <223> séquence exprimant la séquence PKTLS du TYMV (d'après MATSUDA and DREHER, 2004, virology, 321, 36-46)
<220>
   <221> misc_feature
   <222> (196)..(278)
   <223> séquence exprimant le *cis*-ribozyme du HDV (CHDV) (d'après PERROTTA and BEEN, 1991, Nature, 350, 434-436)
<400> 1
   ccaataccct gatgagcttt tgcgaaatag cagctagcat tgaaatagca ttcaatgctc     60
   atactgtgaa cctacacact tccacctaag ttctcgatct ttaaaatcgt tagctcgcca    120
   gttagcgagg tctgtcccca cacgacagat aatcgggtgc aactcccgcc cctcttccga    180
   gggtcatcgg aaccagggtc ggcatggcat ctccacctcc tcgcggtccg acctgggcat    240
   ccgaaggagg acgtcgtcca ctcggatggc taagggag                            278
<210> 2
   <211> 195
   <212> RNA
   <213> Artificial
<220>
   <223> ARN recombinant
<220>
   <221> misc_feature
   <222> (1)..(33)
   <223> trans-ribozyme dirigé contre l'ARNm atp9 (tRzatp9)
<220>
   <221> misc_feature
   <222> (34)..(75)
   <223> séquence de liaison (L)
<220>
   <221> misc_feature
   <222> (76)..(195)
   <223> séquence PKTLS du TYMV (d'après MATSUDA and DREHER, 2004, Virology, 321, 36-46)
<400> 2
   ccaauacccu gaugagcuuu ugcgaaauag cagcuagcau ugaaauagca uucaaugcuc 60
   auacugugaa ccuacacacu uccaccuaag uucucgaucu uuaaaaucgu uagcucgcca 120
   guuagcgagg ucugucccca cacgacagau aaucgggugc aacucccgcc ccucuuccga 180
   gggucaucgg aacca 195
<210> 3
   <211> 25
   <212> DNA
   <213> Artificial
<220>
   <223> amorce directe
<400> 3
   catcggaacc agggtcggca tggca 25
<210> 4
   <211> 23
   <212> DNA
   <213> Artificial
<220>
   <223> amorce réverse
<400> 4
   ggtctctaga ctcccttagc cat 23
<210> 5
   <211> 23
   <212> DNA
   <213> Artificial
<220>
   <223> amorce directe
<400> 5
   gacggatccc ccgcatcgac ctg 23
<210> 6
   <211> 293
   <212> RNA
   <213> Arabidopsis thaliana
<220>
   <221> misc_feature
   <222> (235)..(237)
   <223> triplet ciblé par le trans-ribozyme tRzatp9
<400> 6
   uugucgagau ucaguugguc uucagucuac cacuccgugg guauaagauc gcaaagaaug 60
   cauuccaagu gagaugucca agaucaaagg aacgagggua agaaucgacg aggaaucaau 120
   aagauauaag auaagugaau gacaaagcgu gaguauaauu cucaacccga gauguuagaa 180
   ggugcaaaau caauaggugc cggagcugcu acaauugcuu cagcgggagc ugcuaucggu 240
   auuggaaacg uauucaguuc uuugauucau ucuguggcgc gaaauccauc auu 293
<210> 7
   <211> 58
   <212> DNA
   <213> Artificial
<220>
   <223> amorce directe
<220>
   <221> promoter
   <222> (12)..(36)
   <223> promoteur de l'ARN polymérase du phage T7
<400> 7
   agcaagaatt cgaattgtaa tacgactcac tataggttgt cgagattcag ttggtctt 58
<210> 8
   <211> 32
   <212> DNA
   <213> Artificial
<220>
   <223> amorce réverse
<400> 8
   gtacagaatt caatgatgga tttcgcgcca ca 32
<210> 9
   <211> 85
   <212> DNA
   <213> Artificial
<220>
   <223> amorce
<400> 9
   atgagctttt gcgaaatagc agctagcatt gaaatagcat tcaatgctca tactgtgaac 60
   ctacacactt ccacctaagt tctcg 85
<210> 10
   <211> 19
   <212> DNA
   <213> Artificial
<220>
   <223> amorce
<400> 10
   tggttccgat gaccctcgg 19
<210> 11
   <211> 69
   <212> DNA
   <213> Artificial
<220>
   <223> amorce
<220>
   <221> promoter
   <222> (12)..(36)
   <223> promoteur de l'ARN polymérase du phage T7
<400> 11
   agcaagaatt cgaattgtaa tacgactcac tataggccaa taccctgatg agcttttgcg 60
   aaatagcag 69
<210> 12
   <211> 33
   <212> RNA
   <213> Artificial
<220>
   <223> séquence codant tRzatp9
<400> 12
   ccaauacccu gaugagcuuu ugcgaaauag cag 33
<210> 13
   <211> 107
   <212> DNA
   <213> Artificial
<220>
   <223> amorce
<220>
   <221> misc_feature
   <222> (45)..(86)
   <223> séquence codant pour la séquence de liaison
<400> 13
   gtacaaagct tccaataccc tgatgagctt ttgcgaaata gcagctagca ttgaaatagc 60
   attcaatgct catactgtga acctacacac ttccacctaa gttctcg 107
<210> 14
   <211> 31
   <212> DNA
   <213> Artificial
<220>
   <223> amorce
<400> 14
   agcaagaatt cctcccttag ccatccgagt g 31
<210> 15
   <211> 20
   <212> DNA
   <213> Artificial
<220>
   <223> amorce
<400> 15
   tggttccgat gaccctcgga 20
<210> 16
   <211> 22
   <212> DNA
   <213> Artificial
<220>
   <223> amorce
<400> 16
   ccaataccct gatgagcttt tg 22
<210> 17
   <211> 20
   <212> DNA
   <213> Artificial
<220>
   <223> amorce
<400> 17
   ctcccttagc catccgagtg 20
<210> 18
   <211> 46
   <212> DNA
   <213> Artificial
<220>
   <223> amorce directe
<400> 18
   atgaattcga attgtaatac gactgactat agggcatttg gtctag 46
<210> 19
   <211> 24
   <212> DNA
   <213> Artificial
<220>
   <223> amorce réverse
<400> 19
   caggatcctg gggggcattc cgag 24
<210> 20
   <211> 29
   <212> DNA
   <213> Artificial
<220>
   <223> amorce directe
<400> 20
   gaattcggct aggtaacata atggaaatg 29
<210> 21
   <211> 24
   <212> DNA
   <213> Artificial
<220>
   <223> amorce réverse
<400> 21
   ggatccaggc caaggacggg gtcg 24
<210> 22
   <211> 21
   <212> DNA
   <213> Artificial
<220>
   <223> amorce directe
<400> 22
   acacttccac ctaagttctc g 21
<210> 23
   <211> 45
   <212> DNA
   <213> Artificial
<220>
   <223> amorce réverse
<220>
   <221> promoter
   <222> (1)..(26)
   <223> promoteur de l'ARN polymérase du phage T7
<400> 23
   gaattgtaat acgactcact atagggtggt tccgatgacc ctcgg 45
<210> 24
   <211> 47
   <212> DNA
   <213> Artificial
<220>
   <223> amorce directe
<220>
   <221> promoter
   <222> (1)..(26)
   <223> promoteur de l'ARN polymérase du phage T7
<400> 24
   gaattgtaat acgactcact atagggacac ttccacctaa gttctcg 47
<210> 25
   <211> 23
   <212> DNA
   <213> Artificial
<220>
   <223> amorce directe
<400> 25
   catttggtct agtggtatga ttc 23
<210> 26
   <211> 46
   <212> DNA
   <213> Artificial
<220>
   <223> amorce réverse
<220>
   <221> promoter
   <222> (1)..(26)
   <223> promoteur de l'ARN polymérase du phage T7
<400> 26
   gaattgtaat acgactcact atagggtggg gggcattccg agaatc 46
<210> 27
   <211> 49
   <212> DNA
   <213> Artificial
<220>
   <223> amorce
<220>
   <221> promoter
   <222> (1)..(26)
   <223> promoteur de l'ARN polymérase du phage T7
<400> 27
   gaattgtaat acgactcact atagggcatt tggtctagtg gtatgattc 49
<210> 28
   <211> 20
   <212> DNA
   <213> Artificial
<220>
   <223> amorce réverse
<400> 28
   tggggggcat tccgagaatc 20
<210> 29
   <211> 21
   <212> DNA
   <213> Artificial
<220>
   <223> amorce
<400> 29
   aggttcacgt atgagcattg a 21
<210> 30
   <211> 27
   <212> DNA
   <213> Artificial
<220>
   <223> amorce
<400> 30
   atgctatttc aatgctagct gctattt 27
<210> 31
   <211> 26
   <212> DNA
   <213> Artificial
<220>
   <223> amorce
<400> 31
   atctttaaaa tcgttagctc gccagt 26
<210> 32
   <211> 225
   <212> RNA
   <213> Nicotiana tabacum
<220>
   <221> misc_feature
   <222> (64)..(66)
   <223> triplet cible de l'ARN tRzatp9-L-PKTKS
<400> 32
   auguuagaag gugcaaaauu aaugggugca ggagcugcua caauugcuuu agcgggagcu 60
   gcuaucggua uuggaaacgu uuuuaguucu uugauucauu ccguggcgcg aaauccauca 120
   uuggcaaaac aauuauuugg uuaugccauu uugggcuuug cucuaaccga agcuauugca 180
   uuguuugccc uaaugauggc cuuuuugauc uuauucguau ucuaa 225
<210> 33
   <211> 23
   <212> DNA
   <213> Artificial
<220>
   <223> amorce
<400> 33
   gcaaacgatg caatagcttc ggt 23
<210> 34
   <211> 22
   <212> DNA
   <213> Artificial
<220>
   <223> amorce
<400> 34
   tgctacaatt gcttcagcgg ga 22
<210> 35
   <211> 23
   <212> DNA
   <213> Artificial
<220>
   <223> amorce directe
<400> 35
   cgaagaattg catgaggaag ggc 23
<210> 36
   <211> 21
   <212> DNA
   <213> Artificial
<220>
   <223> amorce
<400> 36
   gccagtggcc gtacaacagg t 21
<210> 37
   <211> 21
   <212> DNA
   <213> Artificial
<220>
   <223> amorce directe
<400> 37
   cttgcccgct tccaattgat g 21
<210> 38
   <211> 20
   <212> DNA
   <213> Artificial
<220>
   <223> amorce
<400> 38
   ctgccaagcc gataggcgaa 20
<210> 39
   <211> 27
   <212> DNA
   <213> Artificial
<220>
   <223> amorce
<400> 39
   ctaacggact tagaatacga ataagat 27
<210> 40
   <211> 193
   <212> RNA
   <213> Artificial
<220>
   <223> ARN recombinant
<220>
   <221> misc_feature
   <222> (1)..(33)
   <223> trans-ribozyme dirigé contre l'ARN mnc1 (tRzmnc1)
<220>
   <221> misc_feature
   <222> (34)..(73)
   <223> séquence de liaison (L)
<220>
   <221> misc_feature
   <222> (74)..(193)
   <223> séquence PKTLS du TYMV (d'après MATSUDA and DREHER, 2004, virology, 321, 36-46)
<400> 40
   aauccagccu gaugagcuuu ugcgaaacug gccacuugcc uauagcacaa ucuauucaug 60
   aacaauagaa gauacacuuc caccuaaguu cucgaucuuu aaaaucguua gcucgccagu 120
   uagcgagguc uguccccaca cgacagauaa ucgggugcaa cucccgcccc ucuuccgagg 180
   gucaucggaa cca 193
<210> 41
   <211> 294
   <212> RNA
   <213> Arabidopsis thaliana
<220>
   <221> misc_feature
   <222> (236)..(238)
   <223> triplet cible du trans-ribozyme tRzmnc1
<400> 41
   ugcuuugcuc gcuccgacga uucuacauac cggccgaaag agacugagcc cgggcgaagc 60
   caaucacauu gaguuguaga uugacauagu uaaccuucag ugcacuuaua uauaauauau 120
   agucagaguu gaagcugagc guucaccuua gcggcaccuc ugaccucaga ugcauguguu 180
   aagcauauaa cuagcgagcg aaccauacuu cggauaucgg cucggagcaa ggccagucgc 240
   uggauuaaga cuaacaggac cauuccuuau gaaagaccca gcaagagacg ucac 294
<210> 42
   <211> 54
   <212> DNA
   <213> Artificial
<220>
   <223> amorce directe
<220>
   <221> promoter
   <222> (12)..(36)
   <223> promoteur de l'ARN polymérase du phage T7
<400> 42
   agcaagaatt cgaattgtaa tacgactcac tataggtgct ttgctcgctc cgac 54
<210> 43
   <211> 29
   <212> DNA
   <213> Artificial
<220>
   <223> amorce
<400> 43
   gtacagaatt cgtgacgtct cttgctggg 29
<210> 44
   <211> 81
   <212> DNA
   <213> Artificial
<220>
   <223> amorce
<400> 44
   gagcttttgc gaaactggcc acttgcctat agcacaatct attcatgaac aatagaagat 60
   acacttccac ctaagttctc g 81
<210> 45
   <211> 69
   <212> DNA
   <213> Artificial
<220>
   <223> amorce
<220>
   <221> promoter
   <222> (12)..(36)
   <223> promoteur de l'ARN polymérase du phage T7
<400> 45
   agcaagaatt cgaattgtaa tacgactcac tataggaatc cagcctgatg agcttttgcg 60
   aaactggcc 69
<210> 46
   <211> 33
   <212> RNA
   <213> Artificial
<220>
   <223> trans-ribozyme tRzmnc1
<400> 46
   aauccagccu gaugagcuuu ugcgaaacug gcc 33
<210> 47
   <211> 276
   <212> DNA
   <213> Artificial
<220>
   <223> Séquence complète de la construction de gène utilisée pour exprimer et importer un trans-ribozyme dirigé contre l'ARN mitochondrial mnc1 dans les mitochondries in vivo
<220>
   <221> misc_feature
   <222> (1)..(33)
   <223> séquence exprimant le trans-ribozyme dirigé contre l'ARN mnc1 (tRzmnc1)
<220>
   <221> misc_feature
   <222> (34)..(73)
   <223> séquence exprimant la séquence de liaison (L)
<220>
   <221> misc_feature
   <222> (74)..(193)
   <223> séquence exprimant la séquence PKTLS du TYMV (d'après MATSUDA and DREHER, 2004, virology, 321, 36-46)
<220>
   <221> misc_feature
   <222> (194)..(276)
   <223> séquence exprimant le *cis*-ribozyme du HDV (CHDV) (d'après PERROTTA and BEEN, 1991, Nature, 350, 434-436)
<400> 47
   aatccagcct gatgagcttt tgcgaaactg gccacttgcc tatagcacaa tctattcatg     60
   aacaatagaa gatacacttc cacctaagtt ctcgatcttt aaaatcgtta gctcgccagt    120
   tagcgaggtc tgtccccaca cgacagataa tcgggtgcaa ctcccgcccc tcttccgagg    180
   gtcatcggaa ccagggtcgg catggcatct ccacctcctc gcggtccgac ctgggcatcc    240
   gaaggaggac gtcgtccact cggatggcta agggag                              276
<210> 48
   <211> 105
   <212> DNA
   <213> Artificial
<220>
   <223> amorce directe
<400> 48
   gtacaaagct taatccagcc tgatgagctt ttgcgaaact ggccacttgc ctatagcaca     60
   atctattcat gaacaataga agatacactt ccacctaagt tctcg                    105
<210> 49
   <211> 193
   <212> RNA
   <213> Artificial
<220>
   <223> ARN recombinant
<220>
   <221> misc_feature
   <222> (1)..(33)
   <223> trans-ribozyme dirigé contre l'ARN *or78* (tRzor78)
<220>
   <221> misc_feature
   <222> (34)..(73)
   <223> séquence de liaison (L)
<220>
   <221> misc_feature
   <222> (74)..(193)
   <223> séquence PKTLS du TYMV (d'après MATSUDA and DREHER, 2004, virology, 321, 36-46)
<400> 49
   ccuauguccu gaugagcuuu ugcgaaacgu ugugaccagc agcugccagu aacacaccua 60
   cgugcgcugc caaacacuuc caccuaaguu cucgaucuuu aaaaucguua gcucgccagu 120
   uagcgagguc uguccccaca cgacagauaa ucgggugcaa cucccgcccc ucuuccgagg 180
   gucaucggaa cca 193
<210> 50
   <211> 504
   <212> RNA
   <213> solanum tuberosum
<220>
   <221> misc_feature
   <222> (89)..(91)
   <223> triplet cible du trans-ribozyme tRzor78
<400> 50
   ggccgaucuc aagcuggaug agaaggcaaa ccaacccucc gaagaaggaa acuaugacua 60
   auuagaccgu uagugaccaa augacaacgu cgacauagga aaagaaggug agauagggga 120
   uggaagcagg guagugcuaa cucugcauca cgcggcuauu uuggcucuuu ccggagagca 180
   ucaaaucaac cgcuuucauu ugcuuugaga cugacccucu uccaggcgaa aaggggaagg 240
   agcuuuccaa ucgaucacac gcccggcaca agagaaggaa gggcaagaga auaaauaggc 300
   acuggcauau ugacuacuug aaauacggac uauauaagaa aaauggggag ggaaugagaa 360
   gaaagccuau uugcaggcaa caaggauuug cugcuuucuu aguuagaaaa aggaguuacc 420
   uagcuagugg agcgagguag uuuacuugcc uuuccacgau uccuacauug agcucucugu 480
   gccuuacuua uuaugaguug auaa 504
<210> 51
   <211> 54
   <212> DNA
   <213> Artificial
<220>
   <223> amorce directe
<220>
   <221> promoter
   <222> (12)..(36)
   <223> promoteur de l'ARN polymérase du phage T7
<400> 51
   agcaagaatt cgaattgtaa tacgactcac tataggccga tctcaagctg gatg 54
<210> 52
   <211> 35
   <212> DNA
   <213> Artificial
<220>
   <223> amorce réverse
<400> 52
   gtacagaatt cttatcaact cataataagt aaggc 35
<210> 53
   <211> 83
   <212> DNA
   <213> Artificial
<220>
   <223> amorce
<400> 53
   atgagctttt gcgaaacgtt gtgaccagca gctgccagta acacacctac gtgcgctgcc 60
   aaacacttcc acctaagttc tcg 83
<210> 54
   <211> 69
   <212> DNA
   <213> Artificial
<220>
   <223> amorce
<220>
   <221> promoter
   <222> (12)..(36)
   <223> promoteur de l'ARN polymérase du phage T7
<400> 54
   agcaagaatt cgaattgtaa tacgactcac tataggccta tgtcctgatg agcttttgcg 60
   aaacgttgt 69
<210> 55
   <211> 33
   <212> RNA
   <213> Artificial
<220>
   <223> trans-ribozyme tRzor78
<400> 55
   ccuauguccu gaugagcuuu ugcgaaacgu ugu 33
<210> 56
   <211> 276
   <212> DNA
   <213> Artificial
<220>
   <223> séquence complète de la construction de gène utilisée pour exprimer et importer un trans-ribozyme dirigé contre l'ARN mitochondrial *or78* dans les mitochondries in vivo
<220>
   <221> misc_feature
   <222> (1)..(33)
   <223> séquence exprimant le trans-ribozyme dirigé contre l'ARN *or78* (tRzor78)
<220>
   <221> misc_feature
   <222> (34)..(73)
   <223> séquence exprimant la séquence de liaison (L)
<220>
   <221> misc_feature
   <222> (74)..(193)
   <223> séquence exprimant la séquence PKTLS du TYMV (d'après MATSUDA and DREHER, 2004, virology, 321, 36-46)
<220>
   <221> misc_feature
   <222> (194)..(276)
   <223> séquence exprimant le *cis*-ribozyme du HDV (CHDV) (d'après PERROTTA and BEEN, 1991, Nature, 350, 434-436)
<400> 56
   cctatgtcct gatgagcttt tgcgaaacgt tgtgaccagc agctgccagt aacacaccta     60
   cgtgcgctgc caaacacttc cacctaagtt ctcgatcttt aaaatcgtta gctcgccagt    120
   tagcgaggtc tgtccccaca cgacagataa tcgggtgcaa ctcccgcccc tcttccgagg    180
   gtcatcggaa ccagggtcgg catggcatct ccacctcctc gcggtccgac ctgggcatcc    240
   gaaggaggac gtcgtccact cggatggcta agggag                              276
<210> 57
   <211> 105
   <212> DNA
   <213> Artificial
<220>
   <223> amorce directe
<400> 57
   gtacaaagct tcctatgtcc tgatgagctt ttgcgaaacg ttgtgaccag cagctgccag     60
   taacacacct acgtgcgctg ccaaacactt ccacctaagt tctcg                    105
<210> 58
   <211> 213
   <212> RNA
   <213> Artificial
<220>
   <223> ARN recombinant
<220>
   <221> misc_feature
   <222> (1)..(53)
   <223> trans-ribozyme dirigé contre l'ARN or78 (tRzor78TS)
<220>
   <221> misc_feature
   <222> (54)..(93)
   <223> équence de liaison (L)
<220>
   <221> misc_feature
   <222> (94)..(213)
   <223> séquence PKTLS du TYMV (d'après MATSUDA and DREHER, 2004, virology, 321, 36-46)
<400> 58
   gcuaaggcaa acgcuaugcu auguccugau gagucggaaa cgacgaaacg uugacuugcc 60
   uauagcacaa ucuauucaug aacaauagaa gauacacuuc caccuaaguu cucgaucuuu 120
   aaaaucguua gcucgccagu uagcgagguc uguccccaca cgacagauaa ucgggugcaa 180
   cucccgcccc ucuuccgagg gucaucggaa cca 213
<210> 59
   <211> 191
   <212> RNA
   <213> Artificial
<220>
   <223> ARN recombinant
<220>
   <221> misc_feature
   <222> (2)..(31)
   <223> trans-ribozyme dirigé contre l'ARN or78 (tRzor78/6+6)
<220>
   <221> misc_feature
   <222> (32)..(71)
   <223> séquence de liaison (L)
<220>
   <221> misc_feature
   <222> (72)..(191)
   <223> séquence PKTLS du TYMV (d'après MATSUDA and DREHER, 2004, Virology, 321, 36-46)
<400> 59
   guauguccug augagcuuuu gcgaaacguu ggagaagaag cugccaguaa cacaccuacg 60
   ugcgcugcca aacacuucca ccuaaguucu cgaucuuuaa aaucguuagc ucgccaguua 120
   gcgaggucug uccccacacg acagauaauc gggugcaacu cccgccccuc uuccgagggu 180
   caucggaacc a 191
<210> 60
   <211> 216
   <212> RNA
   <213> Artificial
<220>
   <223> ARN recombinant
<220>
   <221> misc_feature
   <222> (1)..(56)
   <223> trans-ribozyme dirigé contre l'ARN or78 (tRzor78TS/8+7)
<220>
   <221> misc_feature
   <222> (57)..(96)
   <223> séquence de liaison (L)
<220>
   <221> misc_feature
   <222> (97)..(216)
   <223> séquence PKTLS du TYMV (d'après MATSUDA and DREHER, 2004, virology, 321, 36-46)
<400> 60
   gcuaaggcaa acgcuaugcc cuauguccug augagucgga aacgacgaaa cguuguacuu 60
   gaauauagca caaucuauuc augaacaaaa gaagauacac uuccaccuaa guucucgauc 120
   uuuaaaaucg uuagcucgcc aguuagcgag gucugucccc acacgacaga uaaucgggug 180
   caacucccgc cccucuuccg agggucaucg gaacca 216
<210> 61
   <211> 82
   <212> DNA
   <213> Artificial
<220>
   <223> amorce directe
<400> 61
   gtcggaaacg acgaaacgtt gacttgccta tagcacaatc tattcatgaa caatagaaga 60
   tacacttcca cctaagttct cg 82
<210> 62
   <211> 88
   <212> DNA
   <213> Artificial
<220>
   <223> amorce directe
<400> 62
   agcaagaatt cgaattgtaa tacgactcac tataggctaa ggcaaacgct atgctatgtc 60
   ctgatgagtc ggaaacgacg aaacgttg 88
<210> 63
   <211> 83
   <212> DNA
   <213> Artificial
<220>
   <223> amorce directe
<400> 63
   gatgagcttt tgcgaaacgt tggagaagaa gctgccagta acacacctac gtgcgctgcc 60
   aaacacttcc acctaagttc tcg 83
<210> 64
   <211> 66
   <212> DNA
   <213> Artificial
<220>
   <223> amorce directe
<400> 64
   agcaagaatt cgaattgtaa tacgactcac tataggtatg tcctgatgag cttttgcgaa 60
   acgttg 66
<210> 65
   <211> 82
   <212> DNA
   <213> Artificial
<220>
   <223> amorce directe
<400> 65
   tcggaaacga cgaaacgttg tacttgaata tagcacaatc tattcatgaa caaaagaaga 60
   tacacttcca cctaagttct cg 82
<210> 66
   <211> 91
   <212> DNA
   <213> Artificial
<220>
   <223> amorce directe
<400> 66
   agcaagaatt cgaattgtaa tacgactcac tataggctaa ggcaaacgct atgccctatg    60
   tcctgatgag tcggaaacga cgaaacgttg t                                   91

## Description

La présente invention est relative à de nouveaux moyens pour importer un ribozyme dans les mitochondries des plantes, et plus particulièrement pour obtenir des plantes possédant une stérilité mâle cytoplasmique.

L'obtention de nouvelles variétés végétales est un processus qui se fait en plusieurs étapes sur de nombreuses années. Que ce soit pour les plantes allogames ou autogames, la sélection variétale fait intervenir un croisement entre deux génotypes parentaux homozygotes A et B ayant des caractères agronomique intéressants (par exemple résistance à des maladies, résistance aux parasites, rendement, etc). Afin d'homogénéiser les croisements et de faciliter la sélection, il faut croiser un parent mâle stérile A (c'est à dire ne produisant pas d'anthères ou de pollen fonctionnel) avec un parent fertile B. Ainsi, un ensemble de plantes mâle-stériles provenant de la même variété parentale A est croisé avec de nombreuses variétés parentales B différentes, afin de rechercher les meilleures combinaisons génétiques. La sélection variétale se fera sur les hybrides FI ainsi obtenus à partir des divers croisements.

Or, la plupart des espèces végétales d'intérêt agronomique sont capables de s'autoféconder, mais peu de lignées végétales sont naturellement mâle-stériles. Il faut donc générer cette propriété. Obtenir des plantes mâle-stériles d'une lignée A peut se faire de deux façons conventionnelles.

La première, et la plus simple, consiste à castrer les plantes. Par exemple, pour le maïs, cette castration est fastidieuse et coûteuse car elle nécessite un très grand nombre de personnes. Une castration à l'aide de machines peut aussi être réalisée, mais elle est souvent moins efficace. Dans le cas du blé, la castration est chimique mais peut entraîner des pertes de rendement à la production de semences, réduisant ainsi la possibilité de sélection. De plus, les gamétocides utilisés possèdent souvent des effets phytotoxiques et sont souvent dangereux pour la santé humaine.

La deuxième technique consiste à provoquer une stérilité mâle cytoplasmique (SMC) afin d'obtenir une lignée A génétiquement mâle stérile. Or, l'obtention de telles variétés par des méthodes de croisement nécessite de nombreuses années de recherche et de sélection. En effet, pour « convertir » une lignée d'intérêt mâle fertile en une lignée mâle stérile A, il faut dans un premier temps la croiser avec une lignée C ne possédant pas de caractères particulièrement intéressants, mais naturellement mâle stérile. Par une série de rétrocroisements (6 en général, à raison de 6 mois à 1 an par rétrocroisement), le fond chromosomique de la lignée C est éliminé, permettant ainsi l'obtention d'une lignée mâle stérile A. C'est cette lignée qui sera alors croisée avec la lignée B dont les caractères sont intéressants.

En outre, ces différentes techniques permettant d'obtenir des lignées mâle-stériles sont très dépendantes de l'espèce végétale considérée, et à l'heure actuelle, il n'existe pas de méthodologie applicable à l'ensemble des plantes d'intérêt agronomique cultivées.

On connait par ailleurs quelques méthodes de génie génétique pour induire la stérilité mâle chez les plantes.

Une de ces méthodes utilise le gène codant pour la ribonucléase BARNASE de *Bacillus amyloliquefaciens* (voir la Demande Internationale WO 96/026283 et la Demande de Brevet EP 1 020 527). Le gène codant la BARNASE peut être placé sous le contrôle d'un promoteur permettant une expression spécifique dans le pollen puis introduit dans le génome nucléaire des cellules de plantes par transformation génétique. L'expression de la BARNASE provoque alors la destruction des ARN dans les grains de pollen en formation, entraînant leur dégénérescence. On parle ici de stérilité mâle nucléaire dans la mesure où le transgène est inséré dans le noyau de la cellule de plante transformée et le phénomène qui en découle ne fait pas intervenir les organelles.

Il est aussi connu que l'importation dans les mitochondries d'une protéine exprimée par une séquence codante non éditée d'un gène mitochondrial de végétaux supérieurs (par exemple le gène codant la protéine ATP9 de blé), à l'aide d'un polypeptide susceptible d'importer une protéine dans les mitochondries, permet d'obtenir des plantes mâle-stériles (Demande Internationale WO 94/018334 ; HERNOULD et al., 1993, P.N.A.S. USA, 90, 2370-2374). Les mitochondries de ces plantes présentent cependant l'inconvénient de comprendre à la fois des protéines naturellement exprimées par les mitochondries et des protéines exprimées par la séquence codante non éditée insérée dans le génome nucléaire, réduisant ainsi l'efficacité de cette technique.

Par ailleurs, l'invalidation d'un ARN mitochondrial des cellules de plantes, tel que l'ARNm *atp9* ou un ARN non codant régulateur, pourrait permettre d'obtenir des plantes présentant une stérilité mâle cytoplasmique (SMC ou « CMS » pour « Cytoplasmic Male Sterility »). Ainsi, par exemple, chez *Brassica napus,* il existe un seul gène dans le génome mitochondrial codant pour la protéine ATP9. Une variété mâle stérile, appelée *B. napus* CMS "Tournefortii-Stiewe" et issue d'une fusion de protoplastes entre *B. napus et B. tournefortii,* a été obtenue. Cette variété possède trois gènes *atp9* (*atp9-1a, atp9-1b* et *atp9-2).* En amont d'*atp9*-2*,* il a été retrouvé une séquence dénommée *orf193. orf193* est co-transcrite avec *atp9*-2*.* Aucun codon stop n'existe entre *orf193* et *atp9-2,* suggérant la production d'une nouvelle protéine pouvant rentrer en compétition avec la protéine ATP9 dans l'assemblage du complexe ATP-synthase (DIETERICH et al., 2003, Mol Genet Genomics, 269, 723-731). En outre, chez le Tournesol, une lignée CMS-PEP1 a été isolée et il a été constaté qu'elle possédait un fragment supplémentaire de 500 nucléotides dans la région 3'UTR du gène *atp9.* Cette séquence additionnelle serait à l'origine de la stérilité mâle cytoplasmique présente chez cette lignée. (DE LA CANAL, 2001, Theor Appl Genet, 102, 1185-1189). Par ailleurs, chez la ciboulette, la stérilité mâle cytoplasmique de la lignée CMS1 est due à la présence d'une séquence additionnelle de 762 paires de bases dans la région 3' de la partie codante du gène *atp9 (*ENGELKE et al., 2002, Theor Appl Genet, 104, 698-702).

Les mitochondries des cellules de plantes possèdent un génome principal d'environ 220 à 740 kilobases, mais qui présente une faible densité de gènes, ainsi que, très souvent, des plasmides indépendants du génome principal. Les mitochondries sont le siège de mécanismes génétiques complexes. Les approches génomique, transcriptomique et protéomique de ces dernières années ont apporté de nombreuses informations sur le rôle et le fonctionnement des mitochondries. Cependant, les investigations restent limitées par l'absence d'outils permettant la transformation des mitochondries dans les cellules de plantes et la manipulation de l'expression génétique mitochondriale. De plus, l'absence d'approches de génétique inverse, et plus particulièrement l'absence de données suggérant un adressage mitochondrial d'un système de type DICER/RISC, ou l'existence dans les organelles d'enzymes qui auraient des activités équivalentes, rendent difficile l'identification de nouveaux gènes et de nouvelles fonctions de régulation dans les vastes régions non attribuées des génomes mitochondriaux de plante.

Lors de précédents travaux, les Inventeurs ont montré que la séquence TLS *(« tRNA-like structure* » ou structure en forme d'ARNt), aminoacylable par la valine, du virus TYMV *(Turnip yellow mosaic virus,* numéro d'accession GI:62218 dans la base de données GENBANK) pouvait servir de vecteur d'importation d'une séquence d'ARN passager constituée des 104 nucléotides situés naturellement en amont de la séquence TLS dans le génome du TYMV. Ils ont ainsi fait exprimer dans une cellule de plante, après transformation génétique, une séquence d'ARN composite (recombinant) comprenant ladite séquence d'ARN passager associée à ladite séquence TLS, la séquence TLS étant elle-même associée à son extrémité 3' à la séquence du *cis*-ribozyme de l'HDV *(Hepatitis Delta virus).* Le ribozyme du HDV a pour fonction de libérer l'extrémité 3' de la séquence TLS après transcription, générant ainsi la séquence TLS associée, à son extrémité 5', à la séquence d'ARN passager.

Val et al. (2006, 6ème Rencontre SifrARN, Rennes 2006) divulgue l'utilisation d'une navette d'import constituée de la séquence PKTLS (pseudo-ARNt) aminocylable par la valine du TYMV pour importer*, in vivo* dans les mitochondries végétales, une séquence passagère allant jusqu'à 150 nucléotides.

Les Inventeurs ont maintenant envisagé une nouvelle approche pour manipuler l'expression génétique dans les mitochondries des cellules de plantes en développant une stratégie antisens basée sur un ribozyme.

Les Inventeurs ont recherché si le remplacement de la séquence non fonctionnelle de 104 nucléotides, décrite ci-dessus, par une séquence fonctionnelle de structure complexe, tel qu'un *trans*-ribozyme*,* permettait de conserver d'une part les fonctionnalités d'import de la séquence TLS, et d'autre part les fonctionnalités catalytiques du ribozyme.

Dans ce but, les Inventeurs ont réalisé une construction génétique (polyribonucléotide) associant la séquence d'un ribozyme à tête de marteau ciblant un ARN mitochondrial à la séquence PKTLS aminoacylable par la valine du TYMV (MATSUDA and DREHER, 2004, Virology, 321, 36-46) et ont constaté que la construction pouvait être importée dans les mitochondries des cellules de plantes et qu'en outre, le ribozyme était fonctionnel, c'est-à-dire qu'il était capable de cliver spécifiquement son ARN-cible dans la mitochondrie.

Les ribozymes à tête de marteau sont certainement ceux qui sont les mieux caractérisés de tous les ribozymes naturels connus. Ils ont été découverts initialement dans les viroïdes et les ARN satellites des virus ; leur clivage en *cis* permet la maturation des transcrits générés par la réplication en cercle roulant (« *rolling circle* »). L'étude des ribozymes à tête de marteau autoclivables en *cis* a permis de définir une structure consensus formée de 3 hélices et un coeur conservé (DE LA PEÑA and FLORES, 2001, J Biol Chem, 276, 34586-34593 ; HAMMANN and LILLEY, 2002, Chembiochem, 3, 690-700) pour le développement des systèmes ribozyme/cible en *trans* (*trans-*ribozymes). Cette structure canonique a longtemps été considérée comme optimale. Il a été ensuite montré que des motifs de stabilisation tertiaire (« *tertiary stabilizing motifs* » ou TSM) additionnels étaient essentiels pour le clivage *in vivo* (DE LA PEÑA et al., 2003, EMBO J, 22, 5561-5570 ; KHVOROVA et al., 2003, Nat Struct Biol, 10, 708-712). C'est ce type de ribozyme à tête de marteau, stabilisé par des interactions tertiaires (« *tertiary stabilized hammerhead* » ou tsHH) qui est actuellement considéré comme présentant la meilleure efficacité *in vivo,* notamment dans des conditions de faible concentration en Mg²⁺ (BURKE and GREATHOUSE, 2005, BMC Biochem, 6, 14 ; HOOGSTRATEN and SUMITA, 2007, Biopolymers, 87, 317-328).

Les inventeurs ont aussi montré, de manière surprenante, que dans la construction décrite ci-dessus, les *trans-*ribozymes à tête de marteau d'environ 30 à 34 nucléotides, sans motif de stabilisation tertiaire, et présentant au niveau de leur tige-boucle II, une boucle constituée de 4 nucléotides (UUUU) et une hélice II constituée de seulement 2 paires de nucléotides (G-C et C-G, de la position 5' vers la position 3') présentaient une meilleure efficacité de clivage de l'ARN-cible que les *trans-*ribozymes à tête de marteau stabilisés par des interactions tertiaires (tsHH), et présentant, au niveau de leur tige-boucle II, une boucle constituée de 4 nucléotides (GAAA) et une hélice II constituée de 4 paires de nucléotides (G-C, U-A, C-G, G-C, de la position 5' vers la position 3').

En conséquence, la présente invention a pour objet un polyribonucléotide (ARN) comprenant, de son extrémité 5' vers son extrémité 3', un *trans*-ribozyme à tête de marteau dirigé contre un ARN mitochondrial de plante et une structure en forme d'ARNt aminoacylable par la valine.

On entend par « structure en forme d'ARNt aminoacylable par la valine », la séquence d'ARN mimant un ARNt (« *tRNA-like structure* », TLS), située en position 3' du génome de certains virus de plantes, capable d'être aminoacylée par la valyl-ARNt synthétase et donc de lier un résidu valine de façon covalente en position 3'. Cette structure est appelée séquence TLS^{val}. La structure en forme d'ARNt aminoacylable par la valine lie aussi l'ATP(CTP):ARNt nucléotidyltransférase. La TLS^{val} sous sa forme valylée est également capable de former un complexe avec le facteur d'élongation EF-1A.GTP. En outre, elle n'est généralement pas reconnue par la RNase P *in vivo.* L'Homme du métier connaît plusieurs séquences TLS^{val} (voir par exemple DREHER, 2009, Virus Research, 139, 217-229). A titre d'exemple non limitatif de structures en forme d'ARNt aminoacylables par la valine on peut citer celles contenues dans le génome de virus appartenant aux genres :
- Tymovirus, plus particulièrement aux virus *Turnip yellow mosaic virus* (TYMV), *Andean potato latent virus* (APLV), *Belladonna mottle virus* (BeMV), *Cacao yellow mosaic virus* (CYMV), *Clitoria yellow vein virus* (CYVV), *Eggplant mosaic virus* (EMV), *Kennedya yellow mosaic virus* (KYMV), *Okra mosaic virus* (OkMV), *Ononis yellow mosaic virus* (OYMV) et *Wild cucumber mosaic virus* (WCMV),
- Furovirus, plus particulièrement au *Soil-borne wheat mosaic virus* (SBWMV),
- Pomovirus, plus particulièrement aux virus *Beet soil-borne virus* (BSBV) et *Potato mop-top virus* (PMTV),
- Pecluvirus, plus particulièrement à l'*Indian peanut clump virus* (IPCV) et
- Tobamovirus, plus particulièrement au *Sunnhemp mosaic virus* (SHMV).

De préférence, ladite structure en forme d'ARNt aminoacylable par la valine est celle contenue dans le génome d'un virus, de préférence d'un Tymovirus, et de préférence encore celle contenue dans le génome du *Turnip yellow mosaic virus* (MATSUDA and DREHER, 2004, Virology, 321, 36-46 et DREHER, 2009, cité ci-dessus).

Optionnellement, le dernier nucléotide situé en position 3' de ladite structure en forme d'ARNt aminoacylable par la valine est une adénine (A). Dans le cas où la séquence TLS^{val} ne reconnaîtrait pas l'ATP(CTP):ARNt nucléotidyltransférase (permettant l'ajout d'une adénine à son extrémité 3'), par exemple lors de son utilisation *in vitro,* l'ajout de cette adénine, de manière à obtenir à l'extrémité 3' de la séquence TLS^{val} le triplet -CCA (cytosine-cytosine-adénine) est indispensable pour l'aminoacylation.

On entend par « ribozyme », une molécule d'ARN catalytique capable de cliver spécifiquement une séquence d'ARN-cible en *cis* ou en *trans* (voir pour revue JAMES and GIBSON, 1998, Blood, 91, 371-382 ; SCOTT, 2007, Curr Opin Chem Biol., 11, 636-643). Le ribozyme peut être naturel ou créé artificiellement (synthétique), notamment pour cibler un ARN mitochondrial cible.

On entend par « *trans*-ribozyme dirigé contre un ARN mitochondrial de plante », un ribozyme capable de reconnaître une séquence-cible contenue dans ledit ARN mitochondrial et de cliver cet ARN mitochondrial.

L'ARN mitochondrial cible peut être tout ARN exprimé dans les mitochondries végétales, en particulier un ARN messager (ARNm) ou un ARN non codant (ARNnc).

Un *trans*-ribozyme à tête de marteau est un ARN capable de cliver un ARN-cible, autre que lui-même. Les *trans-*ribozymes à tête de marteau sont bien connus de l'Homme du métier (HAMMANN and LILLEY, 2002, Chembiochem., 3, 690-700; PERSSON et al., 2002, Chembiochem., 3, 1066-1071 ; PERACCHI, 2004, Rev. Med. Virol., 14, 47-64 ; CITTI and RAINALDI, 2005, Curr. Gene Ther., 5, 11-24). Le complexe *trans-*ribozyme à tête de marteau / substrat est généralement constitué d'une hélice intramoléculaire (hélice II) et de deux hélices intermoléculaires (hélices I et III).

Selon un mode de réalisation préféré de l'invention, ledit *trans*-ribozyme à tête de marteau est constitué de 24 à 100 nucléotides, de préférence de 30 à 40 nucléotides, et de préférence encore de 30 à 35 nucléotides.

Selon un autre mode de réalisation préféré de l'invention, ledit *trans-*ribozyme à tête de marteau présente au niveau de sa tige-boucle II, une boucle constituée de 4 nucléotides (choisis parmi adenosine (A), cytidine (C), guanosine (G) et uridine (U)), identiques ou différents, de préférence 4 uridines (UUUU), et une hélice II constituée de 2 paires de nucléotides, de préférence G-C et C-G.

Selon un mode de réalisation avantageux de l'invention, ledit *trans-*ribozyme à tête de marteau est de type RzGII en référence à l'article de PERSSON *et al.,* 2002 (cité ci-dessus), c'est-à-dire qu'il présente au niveau de sa tige-boucle II, une boucle constituée de 4 uridines (UUUU), et une hélice II constituée de 2 paires de nucléotides, G-C et C-G.

Selon un autre mode de réalisation préféré de l'invention, ledit *trans-*ribozyme à tête de marteau s'hybride à l'ARN mitochondrial cible (le substrat du *trans-*ribozyme) par l'intermédiaire de 3 à 50 nucléotides contigus, de préférence de 5 à 10 nucléotides contigus, de préférence encore 8 nucléotides contigus, en position 5' dudit *trans-*ribozyme (correspondant à l'hélice I du *trans*-ribozyme hybridé à sa séquence nucléotidique cible) et par l'intermédiaire de 3 à 50 nucléotides contigus, de préférence de 5 à 10 nucléotides contigus, de préférence 7 nucléotides contigus, en position 3' dudit *trans-*ribozyme (correspondant à l'hélice III du *trans*-ribozyme hybridé à sa séquence nucléotidique cible).

Selon un mode de réalisation avantageux de l'invention, ledit *trans-*ribozyme et ladite structure en forme d'ARNt aminoacylable par la valine sont séparés par une séquence ARN de liaison (L).

On entend par « séquence ARN de liaison » une séquence d'ARN, d'environ 40 nucléotides, de préférence de 35 à 45 nucléotides, choisie de manière à éviter tout appariement (ou toute hybridation) intramoléculaire entre ledit *trans*-ribozyme et le reste dudit polyribonucléotide, en particulier les séquences TLS^{val} ou PKTLS (voir ci-dessous). Ainsi, la fonction de la séquence ARN de liaison est de diminuer ou d'éviter la formation de structures secondaires alternatives au sein dudit polyribonucléotide, notamment entre ledit *trans*-ribozyme et les séquences TLS^{val} ou PKTLS. Elle permet également d'écarter ledit *trans*-ribozyme des séquences TLS^{val} ou PKTLS, afin d'éviter une interférence-ou un effet d'encombrement stérique qui nuirait à la fonctionnalité (inhiberait ou diminuer la fonction) de ces différents composants. La séquence ARN de liaison ne doit pas non plus former, elle-même, de structures secondaires conduisant à un encombrement stérique qui nuirait à la fonctionnalité dudit *trans*-ribozyme ou des séquences TLS^{val} ou PKTLS. L'Homme du métier peut aisément construire une séquence ARN de liaison telle que définie ci-dessus en utilisant un logiciel de prédiction de structures secondaires. A titre d'exemple non limitatif de logiciel, l'Homme du métier peut utiliser le logiciel MFOLD (ZUKER, 1989, Science 244, 48-52 ; ZUKER, 2003, Nucleic Acids Res. 31, 3406-3415 et MATHEWS et al., 1999, J Mol. Biol. 288,911-940).

Selon un autre mode de réalisation avantageux de l'invention, ledit *trans-*ribozyme ou ladite séquence ARN de liaison si elle est présente, et ladite structure en forme d'ARNt aminoacylable par la valine sont séparés par une structure en pseudo-noeud *(« upstream pseudoknot* », UPSK). Cette structure en pseudo-noeud permet d'optimiser l'interaction de la structure en forme d'ARNt aminoacylable par la valine avec l'aminoacyl-ARNt synthétase.

A titre d'exemples non limitatifs de structures en pseudo-noeud utilisables dans le cadre de la présente invention, on peut citer celles naturellement situées en position 5' d'une séquence TLS, de préférence d'une séquence TLS^{val}, contenue dans le génome d'un virus, de préférence appartenant aux genres Tymovirus, Furovirus, Pomovirus, Pecluvirus ou Tobamovirus tels que définis ci-dessus.

De préférence encore, ladite structure en pseudo-noeud est celle qui est contenue dans le génome d'un Tymovirus, préférentiellement celle contenue dans le génome du *Turnip yellow mosaic virus* (MATSUDA and DREHER, 2004, Virology, 321, 36-46).

La séquence constituée, de l'extrémité 5' vers 3', d'une structure en pseudo-noeud et d'une structure en forme d'ARNt est appelée séquence PKTLS. La séquence PKTLS aminoacylable par la valine (PKTLS^{val}) du TYMV a été décrite par MATSUDA and DREHER, 2004 (cité ci-dessus).

Selon un autre mode de réalisation avantageux de l'invention, ladite structure en forme d'ARNt aminoacylable par la valine est associée en position 3' à un ribozyme autoclivable en *cis* en position 5' (dénommé *cis*-ribozyme). La coupure autocatalytique du *cis*-ribozyme permet de libérer l'extrémité 3' (-CC ou -CCA) de la structure en forme d'ARNt aminoacylable par la valine.

Le *cis*-ribozyme est particulièrement avantageux lorsque le génome nucléaire d'une cellule de plante est transformé avec un polydésoxyribonucléotide exprimant, par une ARN polymérase II, un polyribonucléotide composite comprenant un polyribonucléotide tel que défini ci-dessus. Ainsi, dans les cellules transformées, le polyribonucléotide est exprimé dans le noyau par l'ARN polymérase II, générant un polyribonucléotide composite coiffé en 5' et polyadénylé (queue polyA) en 3'. Le *cis-*ribozyme s'autoclive alors et s'élimine avec la queue polyA, libérant l'extrémité 3' (-CC ou CCA) de la séquence TLS^{val}. Le polyribonucléotide résultant (comprenant le *trans*-ribozyme et la séquence TLS^{val}) est alors exporté du noyau vers le cytosol, puis importé du cytosol dans les mitochondries grâce à la séquence TLS^{val}. Une fois dans les mitochondries, le *trans-*ribozyme exerce, à son tour, sa fonction de clivage de l'ARN mitochondrial cible.

Des *cis*-ribozymes utilisables dans le cadre de la présente invention sont connus en eux mêmes de l'Homme du métier (voir pour revue ROSSI, 2007, Proc Natl Acad Sci U S A., 104, 14881-14882). A titre d'exemples non limitatifs, on peut citer les *cis-*ribozymes issus du génome d'un virus, tel que l'*Hepatitis Delta virus* (HDV) (PERROTTA and BEEN, 1991, Nature, 350, 434-436) ou d'un viroïde (DAROS et al., 2006, EMBO Rep, 7, 593-598).

De préférence le *cis*-ribozyme est issu du génome d'un virus, et de préférence encore d'un *Hepatitis Delta virus.*

A titre d'exemple non-limitatif d'ARN messager mitochondrial qui peut être ciblé par un *trans*-ribozyme inclus dans une séquence d'ARN composite conforme à l'invention, on peut citer l'ARNm du gène *atp9* de plante. Le clivage de cet ARNm a pour effet d'induire, chez une plante, une stérilité mâle cytoplasmique.

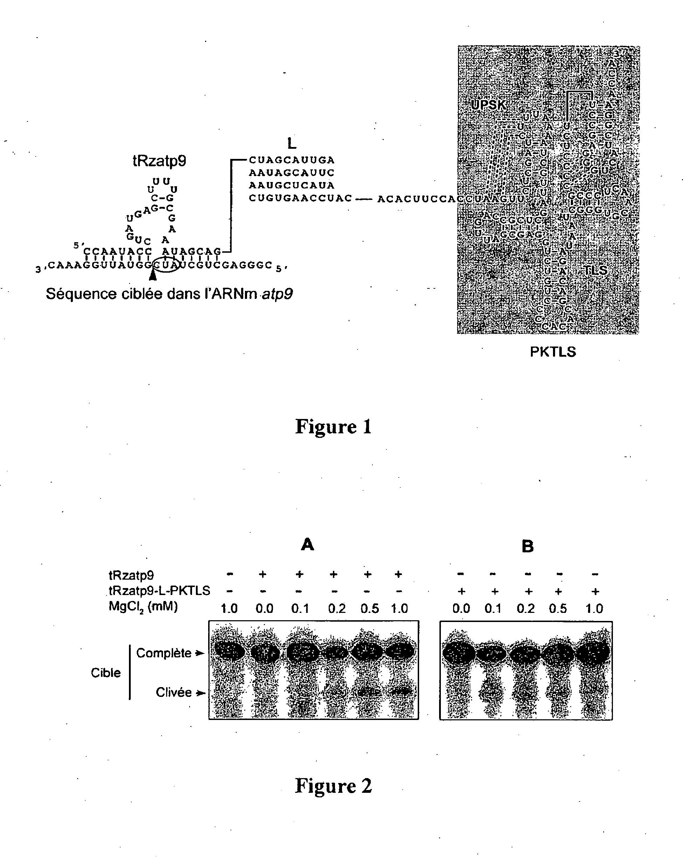

Ainsi, un exemple particulier de polyribonucléotide selon la présente invention constitué d'un *trans*-ribozyme dirigé contre l'ARNm du gène mitochondrial *atp9,* une séquence de liaison et la séquence PKTLS issue du virus TYMV est représenté à la Figure 1 et par la séquence SEQ ID NO : 2.

A titre d'exemples non-limitatifs d'ARN non-codants mitochondriaux qui peuvent être ciblés par un *trans*-ribozyme inclus dans une séquence d'ARN composite conforme à l'invention, on citera les ARN *mnc1* (SEQ ID NO : 41) ou *or 78* (SEQ ID NO : 50).

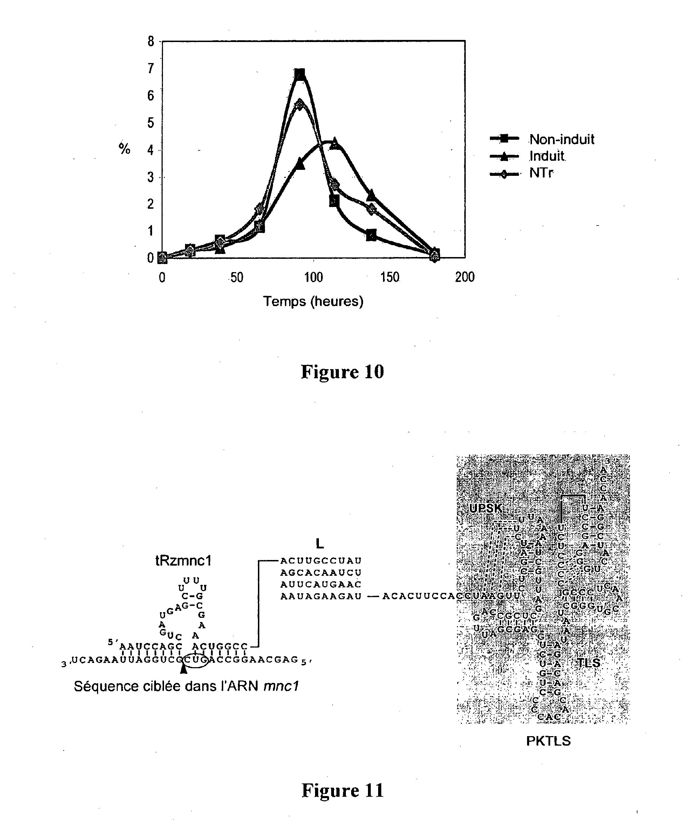

Un exemple de polyribonucléotide selon la présente invention, constitué d'un *trans*-ribozyme dirigé contre l'ARN mitochondrial *mnc1,* une séquence de liaison et la séquence PKTLS issue du virus TYMV, est représenté à la Figure 11 et par la séquence SEQ ID NO : 40.

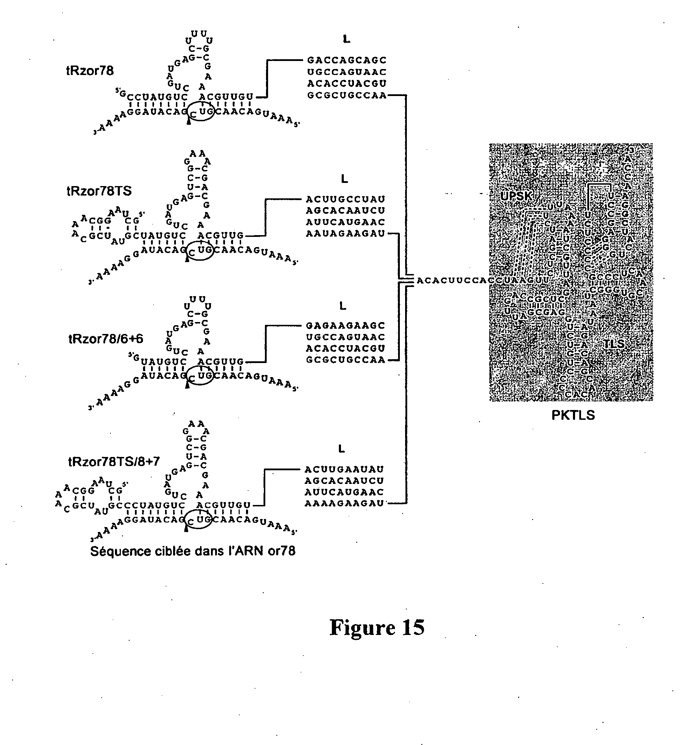

Des exemples de polyribonucléotides selon la présente invention, constitués d'un *trans*-ribozyme dirigé contre l'ARN mitochondrial *or78,* une séquence de liaison et la séquence PKTLS issue du virus TYMV sont représentés à la Figure 15 et par les séquences SEQ ID NO : 49, 58, 59 et 60.

La présente invention a également pour objet l'utilisation d'une structure en forme d'ARNt aminoacylable par la valine (TLS^{val}), de préférence associée à une structure en pseudo-noeud, telles que définies ci-dessus, pour importer dans les mitochondries des cellules de plantes un *trans*-ribozyme à tête de marteau tel que défini ci-dessus.

La présente invention a également pour objet un polydésoxyribonucléotide (ADN) exprimant un polyribonucléotide tel que défini ci-dessus.

Un exemple particulier de polydésoxyribonucléotide selon la présente invention, exprimant un *trans*-ribozyme dirigé contre l'ARNm du gène mitochondrial *atp9,* une séquence de liaison, la séquence PKTLS issue du virus TYMV et le *cis*-ribozyme du virus HDV est représenté par la séquence SEQ ID NO : 1.

Un autre exemple particulier de polydésoxyribonucléotide selon la présente invention exprimant un *trans*-ribozyme dirigé contre l'ARN mitochondrial *mnc1,* une séquence de liaison, la séquence PKTLS issue du virus TYMV et le *cis*-ribozyme du virus HDV, est représenté par la séquence SEQ ID NO : 47.

Un autre exemple particulier de polydésoxyribonucléotide selon la présente invention exprimant un *trans*-ribozyme dirigé contre l'ARN mitochondrial *or78,* une séquence de liaison, la séquence PKTLS issue du virus TYMV et le *cis*-ribozyme du virus HDV, est représenté par la séquence SEQ ID NO : 56.

La présente invention a aussi pour objet une cassette d'expression recombinante, comprenant un polydésoxyribonucléotide tel que défini ci-dessus sous le contrôle d'un promoteur transcriptionnel approprié.

Ledit promoteur transcriptionnel peut être tout promoteur fonctionnel dans une cellule, de préférence une cellule de plante, c'est-à-dire, capable de diriger la transcription d'un polydésoxyribonucléotide tel que défini ci-dessus dans une cellule, de préférence une cellule de plante (voir par exemple pour revue YOSHIDA et SHINMYO, 2000, J Biosci Bioeng., 90, 353-362). Le choix du promoteur le plus approprié dépend notamment de ou des organes ou du ou des tissus ciblé(s) pour l'expression. Le promoteur peut être un promoteur de type constitutif (c'est-à-dire qui est actif dans la plupart des tissus et cellules et dans la plupart des conditions environnementales), spécifique à un type cellulaire (c'est-à-dire qui est actif uniquement ou principalement dans certains tissus ou certains types de cellules) ou inductible (c'est-à-dire, qui est activé par des procédés physiques ou des stimuli chimiques). De préférence, il s'agit d'un promoteur pour l'ARN polymérase II nucléaire.

A titre d'exemples non-limitatifs de promoteurs constitutifs qui sont communément utilisés dans les cellules végétales, on peut citer le promoteur 35S du virus de la mosaïque du chou-fleur (CaMV), le promoteur NOS (nopaline-synthase), le promoteur synthétique PG10-90 (ISHIGE et al., 1999, Plant J., 18, 443-448), de préférence le promoteur 35S.

A titre d'exemples non-limitatifs de promoteurs spécifiques d'un organe ou d'un tissu on peut citer les promoteurs qui sont spécifiques du pollen et/ou des anthères, tels que le promoteur du gène *Zmg13* de maïs (GUERRERO et al., 1990, Mol Gen Genet., 224, 161-168 et HAMILTON et al., 1998, Plant Mol Biol., 38, 663-669), le promoteur du gène *G9* du coton (JOHN and PETERSEN et al., 1994, Plant Mol Biol., 26, 1989-1993), le promoteur Osg6B du soja (TSUCHIYA et al., 1995, Plant Cell Physiol. 36, 487-494), les promoteurs des gènes *OSIPA* et *OSIPK* du riz (GUPTA et al., 2007, Plant Cell Rep., 26, 1919-1931), le promoteur du gène *BcA9* de la navette (LEE et al., 2003, Plant Cell Rep., 22, 268-273), les promoteurs *chi*A P_{A2} et *chi*B P_{B} du pétunia (Van TUNEN et al., 1990, Plant Cell, 2, 393-401), les promoteurs des gènes *LAT52, LAT56* ou *LAT59* de la tomate (TWELL et al., 1991, Genes Dev., 5, 496-507 et EYAL et al., 1995, Plant Cell., 7, 373-384.), le promoteur du gène *g10* du tabac (ROGERS et al., 2001, Plant Mol Biol., 45, 577-585), le promoteur du gène *END1* du pois (GOMEZ et al., 2004, Planta, 219, 967-981 et ROQUE et al., 2007, Plant Cell Rep. 26, 313-325), le promoteur du gène *SLG* de *Brassica* (THORSNESS et al., 1993, Plant Cell., 5, 253-261) et le promoteur chimérique *PSC (*LIU et al., 2008, Plant Cell Rep., 27, 995-1004).

A titre d'exemples non-limitatifs de promoteurs inductibles, on peut citer les systèmes TetR (inductible aux tétracyclines), GVE/VGE (inductible au tébufénozide ou au méthoxyfénozide), GVG, pOp/LhG4 et pOp6/LhGR (inductibles à la déxaméthasone), XVE (inductible à l'oestradiol) (ZUO et al., 2000, Plant J., 24, 265-273), EcR (inductible aux stéroïdes), AlcR (inductible notamment à l'éthanol) (voir pour revue MOORE et al., 2006, Plant J., 45, 651-683 et PADIDAM et al., 2003, Curr. Opin. Plant Biol., 6, 169-177).

Ladite cassette d'expression recombinante comprend aussi un terminateur de transcription, tel que par exemple les terminateurs 35S du CaMV, NOS ou le terminateur T9 du gène rbcS E9 (ZUO *et al.,* 2000, cité ci-dessus; ISHIGE *et al.,* 1999, cité ci-dessus).

La présente invention a aussi pour objet un vecteur recombinant comprenant un polydésoxyribonucléotide ou une cassette d'expression tels que définis ci-dessus.

Les cassettes d'expression et les vecteurs d'expression conformes à l'invention peuvent, bien entendu, comprendre en outre d'autres séquences, usuellement employées dans ce type de constructions, telles que des séquences activatrices de la traduction (TL, « *Translation Leader* »), des sites de polyadénylation, ainsi que, le cas échéant, des séquences amplificatrices (séquences « enhancer » de la transcription). Ils peuvent également comprendre des séquences permettant le suivi de la transformation, ainsi que l'identification et/ou la sélection des cellules ou organismes transformés. Il s'agit notamment de gènes rapporteurs (par exemple celui de la beta-glucuronidase (GUS), celui de la luciférase, ou celui de la *"green fluorescent protein"* (GFP)), conférant à ces cellules ou organismes un phénotype aisément reconnaissable, ou bien de gènes marqueurs de sélection (par exemple des gènes de résistance à un antibiotique tels que la kanamycine ou l'hygromycine, ou à un herbicide).

Le choix du promoteur et des séquences additionnelles pouvant être insérées dans les cassettes et les vecteurs d'expression conformes à l'invention, ainsi que celui du vecteur-hôte, peuvent être effectués, de manière classique, par l'Homme du métier en fonction notamment de critères tels que les cellules et organismes hôtes choisis, le profil d'expression souhaité dans la cellule ou l'organisme hôte, les protocoles de transformation génétique envisagés, etc.

La présente invention englobe également une cellule hôte comprenant une cassette d'expression ou un vecteur recombinant tels que définis ci-dessus.

Les cellules hôtes peuvent être des cellules procaryotes ou eucaryotes. Dans le cas de cellules procaryotes, il peut notamment s'agir d'Agrobactéries telles *qu'Agrobacterium tumefaciens* ou *Agrobacterium rhizobium.* Dans le cas de cellules eucaryotes, il peut s'agir notamment de cellules de plantes, issues de plantes dicotylédones ou monocotylédones, telles que par exemple des cellules du tabac.

La présente invention a également pour objet des plantes génétiquement transformées par au moins un polydésoxyribonucléotide ou une cassette d'expression conforme à l'invention, et notamment des plantes transgéniques comprenant dans leur génome nucléaire au moins une copie d'un transgène contenant un polydésoxyribonucléotide conforme à l'invention.

On définit ici comme plante transgénique une plante transformée chez laquelle l'information génétique exogène apportée par le polydésoxyribonucléotide transformant est intégrée de manière stable dans l'ADN chromosomique, sous forme de transgène, et peut ainsi être transmise aux descendants de ladite plante. Cette définition englobe donc également les descendants des plantes résultant de la transgénèse initiale, dès lors qu'ils contiennent dans leur génome une copie du transgène.

Différentes méthodes d'obtention de plantes transgéniques sont bien connues en elles-mêmes de l'Homme du métier. Généralement, ces méthodes impliquent la transformation de cellules de plantes, la régénération de plantes à partir des cellules transformées, et la sélection des plantes ayant intégré le transgène.

Des techniques très nombreuses de transformation de cellules de plantes germinales ou somatiques (isolées, sous forme de cultures de tissus ou d'organe, ou sur la plante entière), et de régénération des plantes sont disponibles. Le choix de la méthode la plus appropriée dépend généralement de la plante concernée.

Le matériel végétal (protoplastes, cellules, cals, feuilles, boutures, graines, etc...) obtenu à partir des cellules transformées ou des plantes transgéniques conformes à l'invention fait également partie de l'objet de la présente invention. L'invention englobe également les produits obtenus à partir des plantes transgéniques conformes à l'invention, notamment le fourrage, le bois, les feuilles, les tiges, les racines, les fleurs et les fruits.

La présente invention a également pour objet un procédé d'obtention d'une plante transgénique possédant une stérilité mâle cytoplasmique, comprenant les étapes suivantes :
a) obtenir une cellule de plante comprenant une cassette d'expression comprenant une séquence exprimant un ribozyme à tête de marteau dirigé contre l'ARNm *atp9,* l'ARN *mnc1* ou l'ARN *or78,* telle que définie ci-dessus, de préférence dirigé contre l'ARNm *atp9, et*
b) régénérer à partir de la cellule de plante obtenue à l'étape a) une plante transgénique exprimant ledit ribozyme à tête de marteau dirigé contre l'ARNm *atp9,* l'ARN *mnc1* ou l'ARN *or78.*

La présente invention englobe également une plante transgénique susceptible d'être obtenue par le procédé décrit ci-dessus.

La présente invention s'applique aux plantes dicotylédones ou monocotylédones. A titre d'exemples non limitatifs, elle peut s'appliquer aux plantes de la famille des Poacées, Papillonacées, Crucifères, Ombellifères, Solanacées, Légumineuses, Labiées, Astéracées et plus particulièrement au maïs, blé, orge, seigle, triticale, avoine, colza, choux, tabac, pois, tomate, luzerne, betterave, tournesol, soja et riz.

La présente invention sera mieux comprise à l'aide du complément de description qui va suivre, qui se réfère à des exemples non-limitatifs illustrant l'utilisation d'une séquence PKTLS pour importer un *trans*-ribozyme dans les mitochondries des cellules de plantes, ainsi que des figures annexées :
Figure 1 : ARN recombinant (SEQ ID NO : 2) construit pour la stratégie de clivage de l'ARNm *atp9* dans les mitochondries. Le *trans*-ribozyme (tRzatp9) est représenté en interaction avec sa séquence cible. Il est associé à la séquence PKTLS du TYMV (la séquence TLS^{val} correspondant aux 82 derniers nucléotides en position 3') par une séquence de liaison (L). La séquence reconnue par le ribozyme est un triplet AUC (entouré) et le clivage se fait après la cytosine (C) (flèche).
Figure 2 : Activité de clivage du *trans*-ribozyme tRzatp9 seul (A) ou associé à la PKTLS du TYMV dans l'ARN tRzatp9-L-PKTLS (B). L'activité a été déterminée en fonction de la concentration en MgCl₂. Les réactions ont été effectuées pendant 1 heure dans des conditions standard en présence de concentrations croissantes de MgCl₂ et avec un rapport molaire ribozyme/cible de 10/1. Les produits de clivage ont été analysés par électrophorèse sur gel de polyacrylamide.

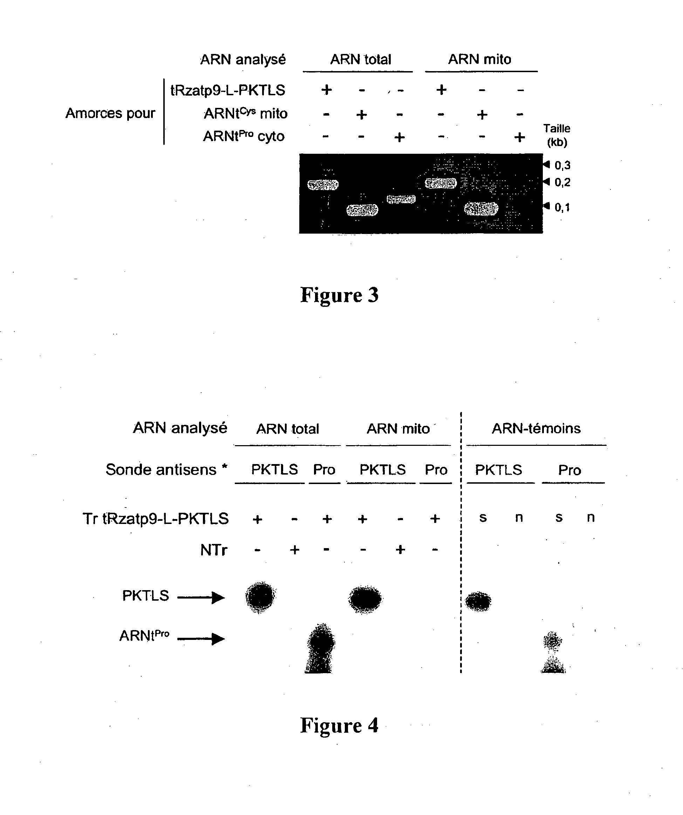

Figure 3 : Analyse par électrophorèse sur gel d'agarose des produits de RT-PCR obtenus à partir d'ARN total ou mitochondrial des cellules de tabac transformées avec le plasmide pER8-tRzatp9LPKTLScHDV et induites à l'oestradiol.
Figure 4 : Analyse par électrophorèse sur gel de polyacrylamide des ARN antisens radioactifs (*) protégés contre les ribonucléases en présence d'ARN total ou mitochondrial des cellules de tabac transformées avec le plasmide pER8-tRzatp9LPKTLScHDV et induites à l'oestradiol. Tr tRzatp9-L-PKTLS = ARN des cellules de tabac transformées ; NTr = ARN des cellules de tabac non transformées. La flèche indique le produit de protection attendu ; Pro = sonde d'ARNt^{Pro} cytosolique ; s = témoin positif de taille résultant de la réaction de protection contre les ribonucléases par un transcrit *in vitro* sens ; n = témoin négatif résultant d'une réaction de protection contre les ribonucléases effectuée avec la sonde antisens seule.

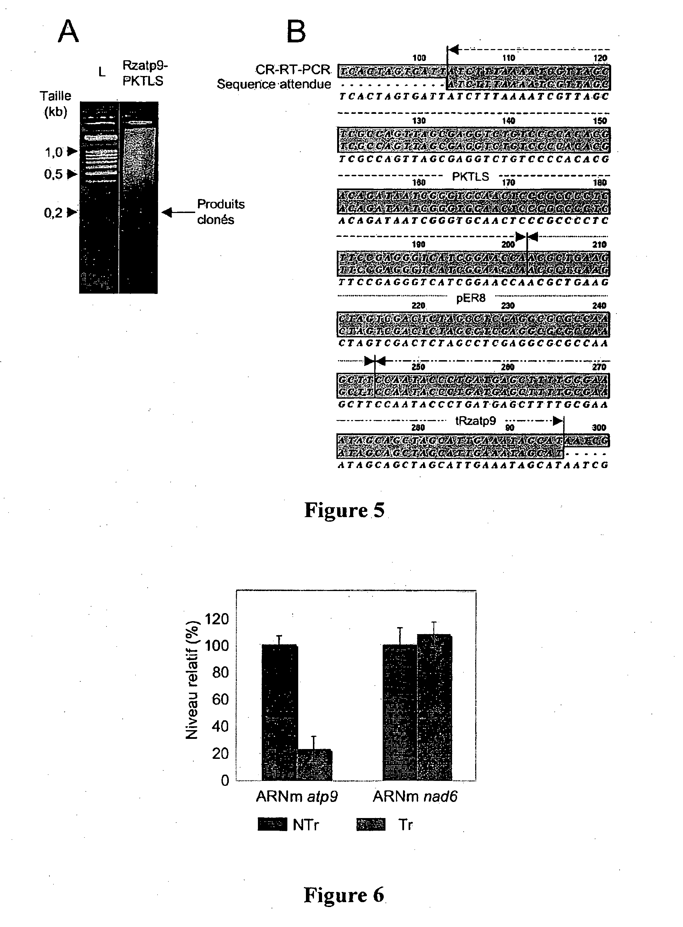

Figure 5 : Analyse des produits de la CR-RT-PCR effectuée après circularisation d'ARN de cellules de tabac transformées avec le plasmide pER8-tRzatp9LPKTLScHDV et induites à l'oestradiol. (A) électrophorèse sur gel d'agarose ; la piste marquée L correspond au marqueur de taille. Seule la PCR nichée finale est représentée. (B) alignement de la séquence des produits de CR-RT-PCR clonés et de la séquence attendue à partir de la construction. PKTLS : partie 5' de la séquence PKTLS du TYMV ; pER8 : région du vecteur pER8 transcrite avec la construction ; tRzatp9 : partie du *trans*-ribozyme.
Figure 6 : Analyse par qRT-PCR du taux des ARNm *atp9* et *nad6* (sous-unité 6 du complexe I de la chaîne respiratoire, NAD(P)H déshydrogénase) dans les ARN extraits de cellules de tabac non transformées (NTr) ou transformées avec le plasmide pER8-tRzatp9LPKTLScHDV (Tr), les deux types de cellules étant induites à l'oestradiol. Les résultats ont été nivelés par rapport à l'ARNm de l'actine et à celui de la protéine ribosomique mitochondriale rp12.

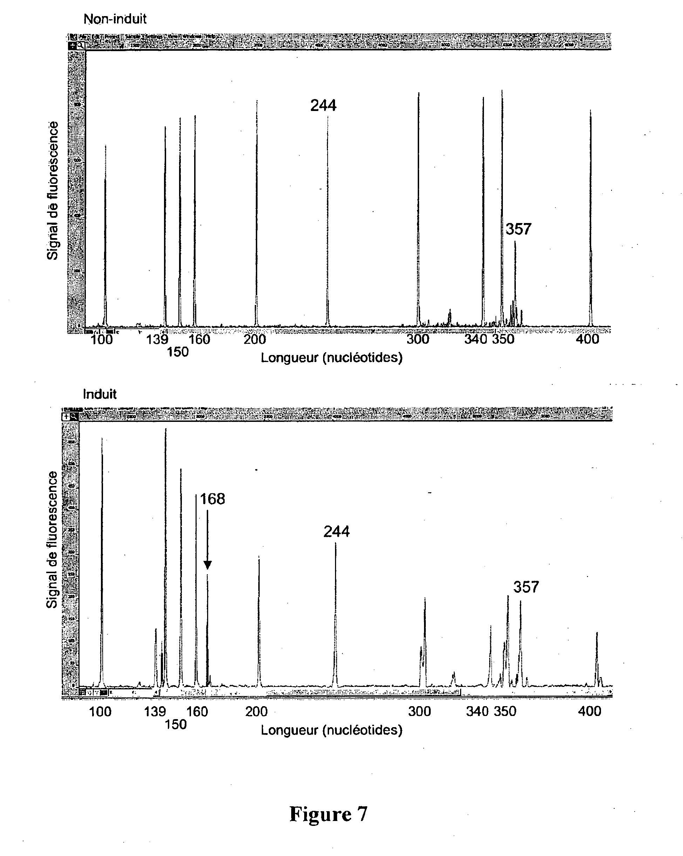

Figure 7 : Analyse par extension d'amorce fluorescente des transcrits *atp9* dans les ARN extraits de cellules de tabac transformées avec le plasmide pER8-tRzatp9LPKTLScHDV et induites ou non induites à l'oestradiol. Le produit de coupure spécifique par le ribozyme tRzatp9 (168 nucléotides) est indiqué par une flèche. Les valeurs en abscisse correspondent à des marqueurs de taille.

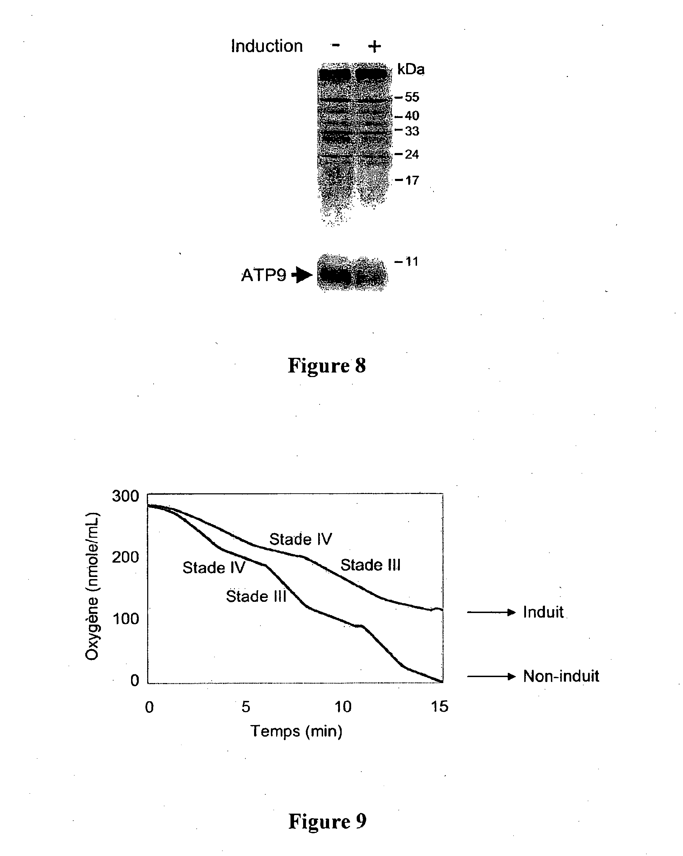

Figure 8 : Analyse sur gel de polyacrylamide des produits de traduction *in organello* en présence de [³⁵S]méthionine dans des mitochondries isolées de cellules de tabac transformées avec le plasmide pER8-tRzatp9LPKTLScHDV et induites ou non induites à l'oestradiol. La migration de la protéine ATP9 est indiquée.
Figure 9 : Mesure, dans un oxymètre, de l'activité respiratoire des mitochondries isolées de cellules de tabac transformées avec le plasmide pER8-tRzatp9LPKTLScHDV et induites ou non induites à l'oestradiol. Stade IV, respiration en l'absence d'ADP; Stade III, respiration après addition d'ADP à une concentration finale de 200 µM.
Figure 10 : Vitesse de croissance des cellules de tabac transformées avec le plasmide pER8-tRzatp9LPKTLScHDV et induites ou non induites à l'oestradiol. NTr = cellules non transformées.
Figure 11 : ARN recombinant (SEQ ID NO : 40) construit pour la stratégie de clivage de l'ARN *mnc1* dans les mitochondries de cellules de plantes. Le *trans*-ribozyme (tRzmnc1) est présenté en interaction avec sa séquence cible. Il est associé à la séquence PKTLS du TYMV par une séquence de liaison (L). La séquence reconnue par le ribozyme est un triplet GUC (entouré) et le clivage se fait après la cytosine (C) (flèche).

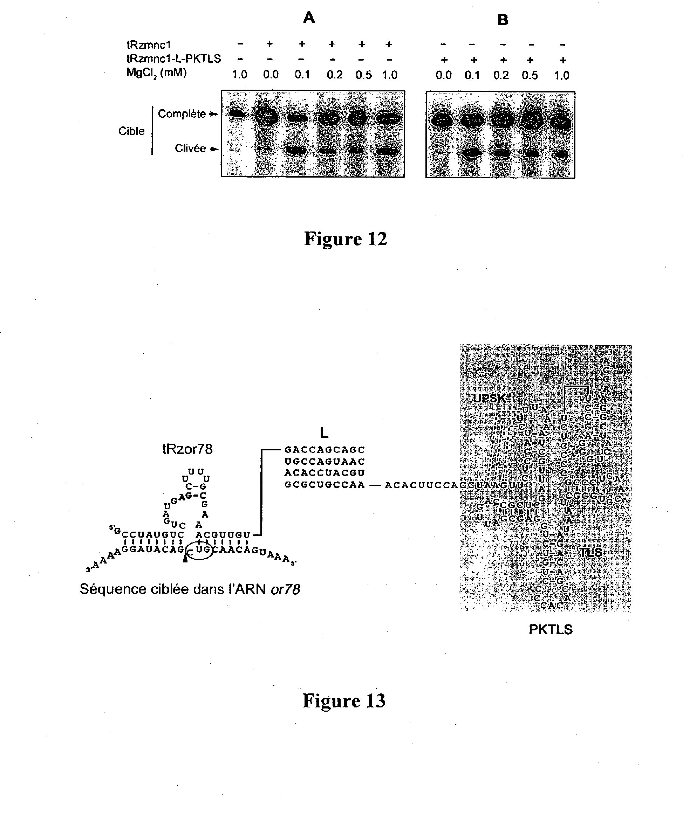

Figure 12 : Activité de clivage du *trans*-ribozyme tRzmnc1 seul (A) ou associé à la PKTLS du TYMV dans l'ARN tRzmnc1-L-PKTLS (B). L'activité a été déterminée en fonction de la concentration en MgCl₂. Les réactions ont été effectuées pendant 1 heure dans des conditions standard en présence de concentrations croissantes de MgCl₂ et avec un rapport molaire ribozyme/cible de 10/1. Les produits de clivage ont été analysés par électrophorèse sur gel de polyacrylamide.
Figure 13 : ARN recombinant (SEQ ID NO : 49) construit pour la stratégie de clivage de l'ARN *or78* dans les mitochondries de cellules de plantes. Le *trans*-ribozyme (tRzor78) est présenté en interaction avec sa séquence cible. Il est associé à la séquence PKTLS du TYMV par une séquence de liaison (L). La séquence reconnue par le ribozyme est un triplet GUC (entouré) et le clivage se fait après la cytosine (C) (flèche).

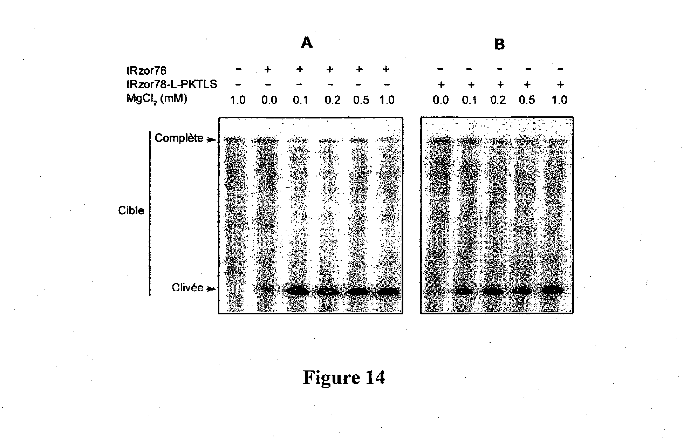

Figure 14 : Activité de clivage du *trans*-ribozyme tRzor78 seul (A) ou associé à la séquence PKTLS du TYMV dans l'ARN tRzor78-L-PKTLS (B). L'activité a été déterminée en fonction de la concentration en MgCl₂. Les réactions ont été effectuées pendant 1 heure dans des conditions standard en présence de concentrations croissantes de MgCl₂ et avec un rapport molaire ribozyme/cible de 10/1. Les produits de clivage ont été analysés par électrophorèse sur gel de polyacrylamide.
Figure 15 : ARN recombinants (SEQ ID NO : 49, 58, 59 et 60) construits pour le clivage de l'ARN *or78* (SEQ ID NO : 50) dans les mitochondries de cellules de plantes. Les *trans-*ribozymes (tRzor78, tRzor78TS, tRzor78/6+6 et tRzor78TS/8+7) sont représentés en interaction avec leur séquence cible. Ils sont associés à la séquence PKTLS du TYMV par une séquence de liaison (L). La séquence reconnue par le ribozyme est un triplet GUC (entouré) et le clivage se fait après la cytosine (C) (flèche).

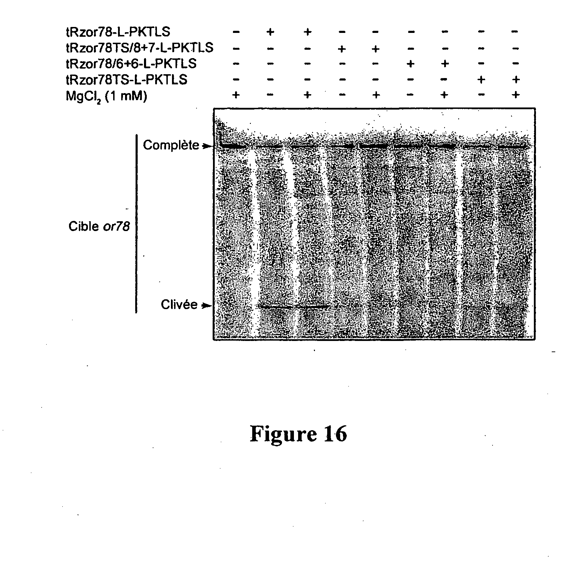

Figure 16 : Activité de clivage des ARN recombinants tRzor78-L-PKTLS (SEQ ID NO : 49), tRzor78TS/8+7-L-PKTLS (SEQ ID NO : 60), tRzor78/6+6-L-PKTLS (SEQ ID NO: 59) et tRzor78TS-L-PKTLS (SEQ ID NO : 58). Les réactions ont été effectuées pendant 1 heure dans des conditions standard en présence ou non de 1 mM de MgCl₂ et avec un rapport molaire ribozyme/ARN-cible de 10/1. Les produits de clivage ont été analysés par électrophorèse sur gel de polyacrylamide.

### EXEMPLE I : UTILISATION DE LA SEQUENCE PKTLS DU TYMV POUR IMPORTER DANS LES MITOCHONDRIES DES CELLULES DE PLANTES UN TRANS-RIBOZYME A TETE DE MARTEAU DIRIGE CONTRE L'ARNm atp9

Une stratégie antisens dans les mitochondries de cellules de plantes basée sur un *trans*-ribozyme à tête de marteau a été développée. Les *trans-*ribozymes à tête de marteau portent leur propre activité de clivage de l'ARN et ne requièrent pas d'autres facteurs. L'ARNm mitochondrial majeur *atp9* codant pour la sous unité 9 de l'ATP synthase a été choisi comme cible à cliver dans les organelles. Sa séquence est hautement conservée d'une espèce végétale à l'autre. La Figure 1 montre les éléments de l'ARN recombinant construit pour cette stratégie. Le *trans*-ribozyme dirigé contre l'ARNm *atp9* (tRzatp9) est associé à la séquence PKTLS du TYMV par l'intermédiaire d'une séquence de liaison (L) à faible potentiel de structuration destinée à éviter les interactions entre les différents éléments de la construction génétique.

### I. MATERIEL & METHODES

### Séquence ARN de liaison (1) :

La séquence L a été élaborée par bioinformatique. Une séquence au hasard de 40 nucléotides a d'abord été définie par ordinateur puis affinée spécifiquement en fonction de chaque séquence-passagère avec l'aide du logiciel de prédiction de structure secondaire MFOLD (MATHEWS et al., 1999, J Mol Biol., 288, 911-940).

### Obtention du plasmide pCK-SPTYPKTLScHDV :

La séquence cHDV a d'abord été amplifiée à partir d'un plasmide recombinant pré-existant pSA1 (PERROTTA et al., 1991, Nature, 350, 434-436) à l'aide de l'amorce directe 5'-CATCGGAACCAGGGTCGGCATGGCA-3' (SEQ ID NO : 3), à cheval sur les séquences à fusionner, et l'amorce réverse 5'-GGTCTCTAGACTCCCTTAGCCAT-3' (SEQ ID NO : 4) portant un site *XbaI* (souligné). Le produit de la première réaction de PCR a été utilisé comme « *megaprimer »* en même temps que l'amorce directe 5'-GACGGATCCCCCGCATCGACCTG-3' (SEQ ID NO : 5) portant un site *BamHI* (souligné) pour obtenir l'ensemble de la construction en prenant comme matrice le plasmide pré-existant pTYMC portant la séquence génomique du TYMV (WEILAND and DREHER, 1989, Nucleic Acids Res., 17, 4675-4687). Le fragment obtenu a ensuite été introduit dans le plasmide pCK-GFPS65C entre les sites *BamHI* et *XbaI* situés en 3' du gène de la GFP, produisant le plasmide pCK-GFPS65C-SPTYPKTLScHDV. Le plasmide pCK-GFPS65C est issu de l'insertion du gène de la GFPS65C (« *green fluorescent protein* ») (HEIM et al., 1995, Nature 373, 663-664) dans le plasmide d'expression pRTL2 (CARRINGTON et al., 1991, Plant Cell, 3, 953-962). Il possède le double promoteur fort 35S du virus de la mosaïque du chou-fleur *(Cauliflower mosaic virus,* CaMV) couplé à une séquence activatrice de la traduction TL *(« Translation Leader* ») du virus de la marbrure du tabac *(Tobacco etch virus,* TEV). En aval du gène codant pour la GFP se trouve le terminateur 35S du CaMV.

Le plasmide pCK-GFPS65C-SPTYPKTLScHDV a été digéré par les enzymes de restriction *XhoI* et *BamHI,* ce qui a permis d'éliminer la séquence TL et le gène de la GFP. Les extrémités cohésives ont été complétées à l'aide du fragment de Klenow de l'ADN polymérase I *d'Escherichia coli,* afin d'obtenir des extrémités franches. Le plasmide a ensuite été recircularisé, générant le plasmide pCK-SPTYPKTLScHDV, les séquences faisant qu'un site de restriction *XhoI* était conservé.

### Transcription de l'ADN in vitro :

Le kit RiboMAX™ (Promega) est utilisé pour les réactions de transcription de l'ADN *in vitro.* Toutes les constructions sont placées sous le contrôle du promoteur de l'ARN polymérase du phage T7. La transcription se déroule pendant 2 heures à 37°C dans un volume réactionnel de 20 µL qui comprend : 2 µg d'ADN linéarisé (issu de PCR ou d'ADN plasmidique), 4 µL de tampon de transcription T7 5X, 6 µL de rNTP (à 25 mM chacun) et 2 µL de la solution T7 mix contenant la polymérase. Pour les transcriptions radioactives, seule la quantité de rNTP varie. Deux microlitres de rNTP (ATP, GTP et CTP à 2,5 mM et UTP à 250 µM) ainsi que 40 µCi d'[α³²P]UTP sont introduits dans la réaction.

Dans les deux cas, après synthèse, 3 unités de DNase dépourvue de RNase sont ajoutées et le volume est complété jusqu'à 50 µL. Une incubation à 37°C pendant 15 min est nécessaire pour digérer l'ADN. Afin d'éliminer les nucléotides non incorporés durant la transcription *in vitro* et les produits de dégradation de l'ADN, le milieu réactionnel est ensuite déposé sur une colonne de Sephadex G-50 d' 1 mL préalablement séchée par centrifugation à 200xg pendant 2 min. L'élution se fait par une nouvelle centrifugation dans les mêmes conditions.

### Tests de clivage in vitro :

Les réactions de clivage ont été effectuées dans les conditions suivantes : 15 nM d'ARN-cible non radioactif et 50 fmoles (30 000 cpm) d'ARN-cible radioactif marqué au [³²P] ont été incubés à 25°C pendant 1-5 heures dans un tampon 50 mM Tris-HCl à pH 7,5 en présence de 150 nM de *trans*-ribozyme tRzatp9 seul ou d'ARN tRzatp9-L-PKTLS. Avant la réaction de clivage, l'ARN-cible et l'ARN catalytique ont été dénaturés pendant 2 min à 75°C puis laissés à refroidir lentement jusqu'à 25°C dans un bloc chauffant (1°C/min). Différentes concentrations de MgCl₂ ont ensuite été ajoutées pour la réaction de clivage (milieu réactionnel final 10 µL). Les produits de clivage ont été séparés par électrophorèse sur gel de polyacrylamide à 8% ou 10% (p/v) en présence d'urée 8 M dans un tampon Tris-borate 90 mM contenant 2 mM d'EDTA (1x TBE). Les gels ont été séchés et soumis à une autoradiographie.

### Transformation des cellules de plantes :

Les bactéries *Agrobacterium tumefaciens* (souche LBA4404) portant la construction d'intérêt dans le plasmide pER8 (ZUO et al., 2000, Plant J., 24, 265-273) sont cultivées dans un milieu LB additionné de MgSO₄ (2 mM), de saccharose (5 g/L) et de rifampicine (25 µg/mL) ou de spectinomycine (100 µg/mL) pendant 2 jours à 28°C sous agitation. Les suspensions de cellules de tabac *(Nicotiana tabacum)* BY-2 (« Bright Yellow 2 » ; NAGATA et al., 1992, Int Rev Cyt., 132, 1-30) sont cultivées à 25°C et à l'obscurité. La veille de leur co-culture avec les cellules de tabac, les bactéries sont repiquées dans un même milieu frais. Le jour même, les bactéries sont reprises dans 1,5 mL de milieu LB. Quatre millilitres d'une culture de cellules de tabac BY-2 de 3 jours sont déposés dans une boîte de Pétri et additionnés de 100 µL de bactéries. Afin de favoriser la transformation des cellules de plante par *A. tumefaciens,* de l'acétosyringone est rajoutée à une concentration de 200 µM. La co-culture est ensuite maintenue à 27°C sous agitation et à l'obscurité pendant 3 jours.

### Sélection des transformants :

Afin d'éliminer au maximum les bactéries présentes, plusieurs lavages successifs avec du milieu de culture des cellules BY-2 sont réalisés. Deux millilitres de suspension cellulaire sont ensuite étalés sur des boîtes contenant du milieu de culture avec de l'agar 0,8% (p/v) et de l'hygromycine (20 µg/mL) pour la sélection des transformants de tabac et de la carbénicilline à 500 µg/mL pour l'élimination des Agrobactéries résiduelles. Les premiers cals résistants apparaissent 2 à 3 semaines après la transformation. Ils sont maintenus sur milieu hygromycine à 25°C à l'obscurité et repiqués sur un milieu frais une fois par mois. Des cultures en suspension liquide maintenant la pression de sélection avec l'hygromycine sont développées à partir de ces cals pour les analyses, en particulier pour la préparation des mitochondries isolées.

### Extraction de l'ARN total :

Deux millilitres de culture de cellules de tabac BY-2 sont prélevés. Après centrifugation pendant 5 min à 14 000xg, les cellules sont resuspendues dans 1 mL de TriReagent^{®} (Molecular Research Center, Inc) et laissées à température ambiante pendant 5 min. Après addition de 200 µL de chloroforme, les échantillons sont agités intensément pendant 15 sec puis laissés à température ambiante pendant 15 min. Une centrifugation de 15 min à 12 000xg permet de séparer la phase aqueuse de la phase phénolique. La phase aqueuse contient les ARN et peu d'ADN. Les ARN sont précipités par addition de 500 µL d'isopropanol et sédimentés par centrifugation pendant 8 min à 12 000xg. Le culot est lavé avec 1 mL d'éthanol à 75% et récupéré par centrifugation à 7 500xg pendant 5 min. Après séchage, les ARN sont redissous dans de l'eau.

### Préparation des mitochondries des cellules de tabac :

Les cultures cellulaires sont filtrées à l'aide d'une trompe à vide. Dix grammes de cellules sont repris doucement à l'aide d'un mortier dans 50 mL de solution enzymatique (pectolyase Y23 1 mg/mL, cellulase RS Onozuka 10 mg/mL, mannitol 0,45 M, MES-KOH 3,6 mM pH 5,5) sans casser les cellules et placés dans une boîte de Pétri. La digestion se fait pendant 2 heures à 30°C, à l'obscurité sous agitation (70 rotations par min). Les protoplastes sont ensuite sédimentés par centrifugation pendant 5 min à 800xg et lavés avec 50 mL de tampon protoplaste (saccaharose 0,3 M, phosphate de potassium 10 mM pH 7,5, EDTA 1 mM, BSA 0,1% p/v, glycine 5 mM). Cette étape de lavage est répétée une deuxième fois.

Une fois lavés, les protoplastes sont repris dans 40 mL de tampon de broyage (saccharose 0,3 M, diphosphate de sodium 30 mM, EDTA 2 mM, BSA 0,3% p/v, PVP 25000 0,8% p/v, pH ajusté à 7,5 avec HCl concentré avant d'ajouter : cystéine 0,05% p/v, glycine 5 mM, ß-mercaptoéthanol 2 mM). Les protoplastes sont éclatés par filtration à travers une toile de nylon (30 µm de vide de maille) sous pression, grâce à une seringue coupée. Les débris cellulaires sont sédimentés par centrifugation pendant 15 min à 2000xg. Les mitochondries contenues dans le surnageant sont sédimentées par une nouvelle centrifugation de 15 min à 11000xg. Le culot de mitochondries est repris dans 500 µL de tampon de lavage (saccharose 0,3 M, phosphate de potassium 10 mM pH 7,5, EDTA 1 mM, BSA 0,1% p/v, glycine 5 mM). Les mitochondries sont ensuite déposées sur un gradient discontinu de Percoll (Sigma) 13,5%, 21% et 45% v/v dans du tampon 2xPB (Tris-HCl 100 mM pH 7,5, saccharose 0,5 M, EDTA 6 mM). Ce gradient est effectué dans des tubes de 2,2 mL contenant 500 µL de Percoll 13,5% et 45% séparés par 600 µL de Percoll 21%. Cent cinquante microlitres de mitochondries reprises dans du tampon de lavage sont déposés sur le gradient. Après centrifugation pendant 20 min à 14 000xg, les mitochondries situées dans la phase à 21 % de Percoll sont prélevées et diluées 10 fois dans du tampon de lavage ne contenant pas de BSA, puis centrifugées pendant 10 min à 12 000xg pour éliminer le Percoll. Cette étape est suivie par une autre étape de lavage/centrifugation dans le même tampon de lavage sans BSA. La quantité de mitochondries est évaluée en mesurant la quantité de protéines par la méthode de Bradford (BRADFORD, 1976, Anal Biochem., 72, 248-254). Les mitochondries peuvent ensuite être utilisées directement pour effectuer des tests de respiration ou pour des tests de synthèse protéique *in organello.*

Si les mitochondries doivent être utilisées pour extraire les ARN, elles sont alors traitées à la RNase afin de limiter les éventuelles contaminations par des ARN cytosoliques. Dans ce cas, le culot brut de mitochondries après la centrifugation de 15 min à 11 000xg est repris dans 500 µL de tampon de lavage contenant une solution de RNases (100 µg/mL de RNase A et 750 U/mL de RNase T1) et mis sous agitation douce pendant 20 min à 25°C. Les mitochondries traitées à la RNase sont ensuite déposées sur un gradient discontinu de Percoll 13,5%, 21% et 45% v/v dans du tampon 2xPB comme décrit ci-dessus. Les lavages successifs se font ensuite avec un tampon de lavage sans BSA contenant de l'EDTA et de l'EGTA 5 mM afin d'inhiber les RNases encore présentes. L'ARN peut être directement extrait du culot final de mitochondries.

### Extraction de l'ARN mitochondrial :

Un à cinq milligrammes de mitochondries sont additionnés de 200 µL de solution d'extraction (Tris-HCl pH 7,5 10 mM, MgCl₂ 10 mM, SDS 1% p/v). L'ensemble est agité vigoureusement 2 min, puis centrifugé 10 min à 10 000x*g*. Le surnageant est extrait au phénol puis les acides nucléiques sont précipités à l'éthanol. Des traitements successifs à la DNase permettent ensuite d'éliminer l'ADN.

### Protocole de RT-PCR :

L'enzyme utilisée pour réaliser la réaction de transcription inverse est la "SuperScript™ III" (Invitrogen). Le protocole utilisé est celui recommandé par le fabricant. Dans un premier temps, 300 ng à 5 µg d'ARN total ou mitochondrial sont ajoutés à 2 pmoles de l'amorce spécifique de l'ARN recherché ou à 250 ng d'un mélange d'hexamères (dans le cas des RT-PCR quantitatives). Un microlitre de dNTP (10 mM chacun) est ajouté puis le volume est complété à 13 µL. Le mélange est incubé 5 min à 65°C puis immédiatement mis sur la glace pendant au moins 1 min. Quatre microlitres de tampon de réaction (250 mM Tris-HCl pH 8,3, 375 mM KCl, 15 mM MgCl₂) sont ensuite ajoutés, ainsi que 1 µL de DTT 0,1 M, 40 unités d'inhibiteur de RNases (RNase out, Invitrogen) et 200 unités d'enzyme SuperScript™ III (200 U/µL dans 20 mM Tris-HCl pH 7.5, 100 mM NaCl, 0.1 mM EDTA, 1 mM DTT, 0.01% Nonidet P-40 v/v, 50% glycerol p/v). Les échantillons sont ensuite placés 5 min à 25°C, puis une heure à 55°C. Afin d'inactiver la réaction, une incubation à 70°C pendant 15 min est réalisée. Les échantillons sont directement utilisés pour la réaction de PCR.

### Tests de protection contre les ribonucléases :

Le protocole de protection contre les ribonucléases est issu de la méthode décrite par GOODALL et al., 1990 (Methods Enzymol., 181, 148-161). Une sonde ARN antisens radioactive est synthétisée par transcription *in vitro.* Après précipitation éthanolique, la sonde est redissoute dans du tampon d'hybridation (80% formamide v/v, 40 mM PIPES pH 6,7, 400 mM NaCl, 1 mM EDTA) et sa radioactivité est comptée. Huit cent nanogrammes d'ARN total ou mitochondrial sont précipités et redissous dans 10 µL de tampon d'hybridation. L'équivalent de 20 000 cpm de sonde antisens y est ensuite ajouté et le volume est complété à 20 µL. Une dénaturation de 10 min à 95°C est nécessaire avant une hybridation à 55°C durant la nuit. La sonde antisens s'hybride alors à l'ARN-cible.

Le lendemain, 200 µL de solution RNase (10 mM Tris-HCl pH 7,5, 5 mM EDTA, 100 mM LiCl, 200 mM NaCl, 100 µg/mL RNase A, 750 u/mL RNase T1) sont ajoutés et les échantillons sont incubés 30 min à 30°C afin d'éliminer les ARN non hybridés et les extrémités non protégées de la sonde antisens radioactive. Puis, afin d'éliminer les RNases A et T1, 6 µL de SDS 10% p/v et 6 µL de protéinase K (10 mg/mL) sont ajoutés. Après 15 min d'incubation à 37°C, les échantillons sont extraits avec un mélange phénol/chloroforme (1/1), précipités à l'éthanol puis resuspendus dans du tampon de dépôt et séparés sur gel de polyacrylamide dénaturant à 8% p/v. Le gel est ensuite séché puis révélé par autoradiographie.

### Protocole de RT-PCR sur ARN circularisé (CR-RT-PCR) :

Une première réaction vise à éliminer la coiffe potentiellement présente à l'extrémité 5' des ARN à analyser. Cette réaction se fait grâce à la pyrophosphatase acide de tabac TAP (pour "Tabacco Acid Pyrophosphatase") (EPICENTRE Biotechnologies). Les ARN sont incubés pendant 30 min à 37°C dans un tampon acétate de sodium 50 mM (pH 6,0), beta-mercaptoéthanol 0,1% v/v, EDTA 1 mM et Triton X-100 0,01% v/v, en présence d'une unité d'enzyme par nanomole d'ARN et d'ATP 2 mM. Les ARN sont ensuite extraits au phénol et précipités à l'éthanol avant d'être circularisés à l'aide de la T4 RNA ligase (Fermentas). Dix microgrammes sont incubés pendant 3 heures à 37°C en présence de 100 unités d'enzyme et de 3,4 µM d'ATP dans du tampon de ligation (50 mM HEPES-NaOH pH 8,0, 10 mM MgCl₂, 10 mM DTT). Une réaction de transcription inverse est ensuite réalisée afin de synthétiser l'ADNc correspondant à la ligation entre les extrémités 5' et 3' de l'ARN analysé. Une réaction de PCR initiale puis une réaction de PCR nichée sont utilisées afin d'amplifier cette séquence puis de la cloner dans le vecteur pGEM®-TEasy (Promega). L'amplicon est ensuite séquencé.

### Protocole de qRT PCR :

Les amorces sont dessinées grâce au logiciel PrimerExpress (Applied Biosystems). Leur efficacité est vérifiée par le logiciel LinRegPCR (bioinfo@amc.uva.nl) (RAMAKERS et al., 2003, Neurosci Lett., 339, 62-66). Toutes les analyses sont opérées grâce au logiciel iQ5 (Biorad). Les réactions de PCR sont réalisées dans un milieu réactionnel de 20 µL, composé de 10 µL de kit SybrGreen (MasterMix Plus, Eurogentec), de 0,6 µM d'amorces directe et retour, ainsi que de 1 µL de réaction de RT (réalisée avec un mélange d'hexamères). Le volume est complété avec de l'eau.

Le programme utilisé est le suivant :

| | |
|---|---|
| Un cycle: | - 95°C, 30 sec |
| Un cycle : | - 50°C, 2 min |
| | - 95°C, 10 min |
| Quarante cycles : | - 95°C, 1 min |
| | - 60°C, 1 min |
| Un cycle: | - 95°C, 1 min |
| | - 55°C, 1 min |

| | |
|---|---|
| Quatre-vingts cycles : | - de 55°C à 94,5°C ; incrémenté de 0,5°C toutes les 10 sec |

Le logiciel geNorm (VANDESOMPELE et al., 2002, Genome Biol., 3, RESEARCH0034) a permis de définir les gènes de l'actine et de la protéine ribosomique mitochondriale rpl2 comme étant les plus stables. Ces gènes ont donc servi de références aux expériences.

### Protocole d'extension d'amorce fluorescence (Fluorescently Labelled Oligonucleotide Extension, FLOE) :

Cinq microgrammes d'ARN mitochondriaux sont utilisés dans une réaction de transcription inverse en utilisant 5 nM d'une amorce marquée en 5' par la molécule fluorescente ROX et complémentaire de l'extrémité 3' codante du gène *atp9.* La réaction se fait dans 30 µL. Une seconde réaction est réalisée par addition de dNTP (1,5 mM), de 40 U de RNase out (Invitrogen), de 25 U d'enzyme Superscript III (Invitrogen) et de tampon 1X. Le volume est complété à 40 µL. Dix microlitres sont ensuite précipités à l'éthanol et resuspendus dans 2 µL d'eau auxquels sont additionnés 8 µL de marqueur de taille fluorescent GeneScan 500 ROX (Applied Biosystems) dilué au millième. Le tout est séparé par électrophorèse capilaire et visualisé grâce au séquenceur de type Applied Biosystems.

### Protocole de synthèse protéique in organello :

Après extraction, une quantité de mitochondries équivalente à 100 µg de protéines est incubée pendant une heure à 25°C dans 100 µL de tampon IS (KH₂PO4 5 mM, mannitol 300 mM, KCl 60 mM, HEPES 50 mM pH 7,0, MgCl₂ 10 mM, Na malate 10 mM, NADH 10 mM), 2 mM de GTP, 4 mM d'ADP, 2 mM de DTT, 0,1% p/v de BSA, 25 µM de chaque acide aminé excepté la méthionine et de 20 µCi de [³⁵S]méthionine. Durant la réaction, la [³⁵S]méthionine s'incorpore aux protéines en cours de synthèse. La réaction est stoppée en ajoutant 1 mL de tampon de lavage (tels que décrit pour la préparation des mitochondries) contenant 10 mM de méthionine non radioactive. Les mitochondries sont ensuite sédimentées par centrifugation pendant 5 min à 12 000xg et conservées à -80°C. L'équivalent de 20 µg de protéines est ensuite séparé sur gel de polyacrylamide et les protéines mitochondriales ainsi marquées durant la réaction sont ensuite révélées en fluorographie.

### II. RESULTATS

### II.1. Validation in vitro de l'activité trans-ribozyme de l'ARN tRzatp9-L-PKTLS

La fonctionnalité du système *trans*-ribozyme/cible a été testée *in vitro* pour vérifier l'activité du ribozyme et la conservation de cette activité après association avec la séquence PKTLS du TYMV.

### 1) Constructions de gènes

L'ARN-cible et l'ARN tRzatp9-L-PKTLS ont été synthétisés par transcription *in vitro* à partir d'un produit PCR. La séquence-cible atp9 (SEQ ID NO : 6) a été amplifiée par PCR à partir d'ADN d'*Arabidopsis thaliana* avec l'amorce directe 5'-AGCAAGAATTCGAATTGTAATACGACTCACTATAGGTTGTCGAGATTCAGTTGGT CTT-3' (SEQ ID NO : 7) portant le promoteur de l'ARN polymérase du phage T7 (souligné) et l'amorce réverse 5'-GTACAGAATTCAATGATGGATTTCGCGCCACA-3' (SEQ ID NO : 8). La matrice ADN pour la synthèse de l'ARN tRzatp9-L-PKTLS a été assemblée par deux réactions de PCR consécutives, la première avec les amorces 5'-ATGAGCTTTTGCGAAATAGCAGCTAGCATTGAAATAGCATTCAATGCTCATACTG TGAACCTACACACTTCCACCTAAGTTCTCG-3' (SEQ ID NO: 9) et 5'-TGGTTCCGATGACCCTCGG-3' (SEQ ID NO : 10), la deuxième avec les amorces 5'-AGCAAGAATTCGAATTGTAATACGACTCACTATAGGCCAATACCCTGATGAGCTT TTGCGAAATAGCAG-3' (SEQ ID NO : 11) (promoteur de l'ARN polymérase du phage T7 souligné) et de séquence SEQ ID NO : 10.

### 2) Tests de clivage in vitro

Afin de pouvoir visualiser son clivage, l'ARN-cible a été synthétisé *in vitro* sous forme radioactive. Il a ensuite été incubé avec l'ARN tRzatp9-L-PKTLS synthétisé à partir de la matrice PCR ou avec un ARN synthétique (Sigma-Aldrich) correspondant strictement à la séquence du *trans*-ribozyme (SEQ ID NO : 12 ; 5'-CCAAUACCCUGAUGAGCUUUUGCGAAAUAGCAG-3'). La réaction a été testée en présence de concentrations de MgCl₂ susceptibles d'être retrouvées *in vivo* (1 à 2 mM) (IGAMBERDIEV and KLECZKOWSKI, 2001, Biochem J., 360, 225-231).

Les résultats sont représentés à la Figure 2. Les tests *in vitro* ont montré que le *trans*-ribozyme tRzatp9, tel qu'il a été conçu, était capable de cliver sa cible dans des conditions de concentrations de MgCl₂ réduites et que son activité était conservée dans les mêmes conditions une fois qu'il était associé à la séquence PKTLS du TYMV par l'intermédiaire de la séquence de liaison spécifique L dans l'ARN tRzatp9-L-PKTLS.

### II.2. Invalidation de l'ARNm atp9 dans les mitochondries des cellules de plantes in vivo

### 1) Construction de gènes et transformation des cellules de plantes

La séquence complète (SEQ ID NO : 1) exprimant le *trans*-ribozyme tRzatp9, la séquence de liaison L, la séquence PKTLS du TYMV et le *cis*-ribozyme cHDV a été assemblée par amplification PCR à partir du plasmide pCK-SPTYPKTLScHDV avec l'amorce directe (SEQ ID NO: 13) 5'-GTACAAAGCTTCCAATACCCTGATGAGCTTTTGCGAAATAGCAGCTAGCATTGAA ATAGCATTCAATGCTCATACTGTGAACCTACACACTTCCACCTAAGTTCTCG-3' (site *HindIII* souligné) et l'amorce réverse 5'-AGCAAGAATTCCTCCCTTAGCCATCCGAGTG-3' (SEQ ID NO: 14) (site *EcoRI* souligné). Le fragment obtenu a été introduit dans le plasmide pUCAP (VAN ENGELEN et al., 1995, Transgenic Res., 4, 288-290) entre les sites *HindIIII* et *EcoRI,* puis ré-excisé avec les enzymes *AscI* et *PacI* et finalement cloné entre les sites *AscI* et *PacI* du plasmide pER8 (ZUO et al., 2000, Plant J., 24, 265-273), générant ainsi le plasmide pER8-tRzatp9LPKTLScHDV. La construction a été ainsi insérée dans le plasmide pER8 dans la cassette d'expression sous le contrôle du promoteur XVE inductible à l'oestradiol.

Le plasmide final pER8-tRzatp9LPKTLScHDV a été utilisé pour transformer des cellules de tabac BY-2 par l'intermédiaire *d'Agrobaclerium tumefaciens.*

### 2) Analyse de l'expression et de l'import dans les mitochondries de l'ARN tRzatp9-L-PKTLS

Des cultures en suspension liquide ont été développées à partir des cals de *N. tabacum* transformés avec le plasmide pER8-tRzatp9LPKTLScHDV. La construction a été laissée silencieuse ou a été induite à l'oestradiol. Des cellules non transformées traitées ou non à l'oestradiol ont également été utilisées comme contrôle. Les suspensions cellulaires ont servi à isoler des mitochondries qui ont été extraites pour préparer de l'ARN mitochondrial. De l'ARN total a d'autre part été préparé à partir des mêmes cellules. L'expression et l'import dans les mitochondries de l'ARN tRzatp9-L-PKTLS ont ensuite été analysés par RT-PCR et par protection d'une sonde antisens radioactive contre les nucléases.

Pour l'analyse par RT-PCR, la réaction de transcription inverse a été effectuée avec l'amorce 5'-TGGTTCCGATGACCCTCGGA-3' (SEQ ID NO: 15) complémentaire à l'extrémité 3' de la séquence PKTLS. Pour la réaction d'amplification par PCR, cette amorce a ensuite été couplée avec l'amorce directe 5'-CCAATACCCTGATGAGCTTTTG-3' (SEQ ID NO : 16) correspondant à l'extrémité 5' de la séquence du ribozyme tRzatp9. Pour vérifier l'efficacité de la coupure autocatalytique du *cis-*ribozyme cHDV, des réactions similaires ont été effectuées avec la même amorce directe (SEQ ID NO : 16) et l'amorce réverse 5'-CTCCCTTAGCCATCCGAGTG-3' (SEQ ID NO : 17) complémentaire à l'extrémité 3' de la séquence cHDV. Afin de tester la contamination résiduelle des échantillons mitochondriaux par des ARN cytosoliques, des réactions de RT-PCR ont été réalisées avec l'amorce directe 5'-ATGAATTCGAATTGTAATACGACTGACTATAGGGCATTTGGTCTAG-3' (SEQ ID NO : 18) et l'amorce réverse 5'-CAGGATCCTGGGGGGCATTCCGAG-3' (SEQ ID NO : 19) permettant d'amplifier l'ARNt^{Pro}(UGG) et l'ARNt^{Pro}(AGG) cytosoliques. Pour caractériser les échantillons d'ARN mitochondriaux, des réactions de RT-PCR ont été réalisées avec des amorces spécifiques de l'ARNt^{Cys}(GCA) mitochondrial en utilisant l'amorce directe 5'-GAATTCGGCTAGGTAACATAATGGAAATG-3' (SEQ ID NO : 20) et l'amorce réverse 5'-GGATCCAGGCCAAGGACGGGGTCG-3' (SEQ ID NO : 21).

Les résultats (Figure 3) ont montré la présence de l'ARN tRzatp9-L-PKTLS dans la fraction d'ARN total et dans la fraction d'ARN mitochondrial provenant des cellules de tabac transformées avec le plasmide pER8-tRzatp9LPKTLScHDV et induites à l'oestradiol. L'identité des produits PCR a été vérifiée par clonage et séquençage. L'amplification des ARNt^{Pro} a montré l'absence de contamination significative de la fraction mitochondriale par des ARN cytosoliques, tandis que l'amplification de l'ARNt^{Cys} a confirmé l'identité de la fraction mitochondriale. Les amplifications PCR de contrôle faisant suite à des réactions de RT-PCR dans lesquelles la transcriptase inverse n'a pas été ajoutée n'ont pas généré de produits. De même, l'absence de produits d'amplification dans les réactions effectuées avec l'amorce réverse complémentaire à l'extrémité 3' de la séquence cHDV a confirmé l'autoclivage efficace du *cis*-ribozyme.

Des tests de protection d'une sonde ARN antisens radioactive contre les ribonucléases ont également été développés. La sonde antisens a été synthétisée par transcription *in vitro* à partir d'un produit PCR. Pour cela, la séquence PKTLS du TYMV a été amplifiée par PCR avec l'amorce directe 5'-ACACTTCCACCTAAGTTCTCG-3' (SEQ ID NO : 22) et une amorce réverse portant le promoteur de l'ARN polymérase du phage T7 (souligné) 5'-GAATTGTAATACGACTCACTATAGGGTGGTTCCGATGACCCTCGG-3' (SEQ ID NO : 23). Afin de servir de contrôle positif, un transcrit sens de la séquence PKTLS du TYMV non radiomarqué a été synthétisé de la même manière à partir d'un produit PCR généré avec l'amorce directe 5'-GAATTGTAATACGACTCACTATAGGGACACTTCCACCTAAGTTCTCG-3' (SEQ ID NO : 24) portant le promoteur T7 (souligné) et l'amorce réverse de séquence SEQ ID NO : 15. Pour tester la contamination cytosolique dans les fractions mitochondriales, des transcrits antisens radioactifs et sens non marqués correspondant aux ARNt^{Pro} cytosoliques ont été synthétisés de la même manière. La matrice PCR pour la séquence ARNt^{Pro} antisens a été générée avec l'amorce directe 5'-CATTTGGTCTAGTGGTATGATTC-3' (SEQ ID NO : 25) et l'amorce réverse portant le promoteur de l'ARN polymérase du phage T7 (souligné) 5'-GAATTGTAATACGACTCACTATAGGGTGGGGGGCATTCCGAGAATC-3' (SEQ ID NO : 26). La matrice PCR pour la séquence ARNt^{Pro} sens a été générée avec l'amorce directe portant le promoteur de l'ARN polymérase du phage T7 (souligné) 5'-GAATTGTAATACGACTCACTATAGGGCATTTGGTCTAGTGGTATGATTC-3' (SEQ ID NO : 27) et l'amorce réverse 5'-TGGGGGGCATTCCGAGAATC-3' (SEQ ID NO : 28).

Les tests ont montré la protection contre les RNases d'un ARN antisens de la taille attendue en présence d'ARN total et d'ARN mitochondrial des cellules de tabac transformées avec le plasmide pER8-tRzatp9LPKTLScHDV et induites à l'oestradiol (Figure 4). Les essais avec les sondes d'ARNt^{Pro} n'ont pas révélé de contamination cytosolique significative.

Les expériences de RT-PCR et de protection contre les ribonucléases ont donc montré l'expression de l'ARN tRzatp9-L-PKTLS et son import *in vivo* dans les mitochondries des cellules de tabac BY-2 transformées et induites à l'oestradiol.

Les extrémités 5' et 3' exactes des transcrits issus de la construction tRzatp9LPKTLScHDV dans les cellules transformées ont été analysées par RT-PCR sur des ARN circularisés (CR-RT-PCR). Après circularisation d'ARN total de cellules transformées, un ADNc couvrant la jonction créée par la circularisation de l'ARN tRzatp9-L-PKTLS a été synthétisé avec l'amorce 5'-AGGTTCACGTATGAGCATTGA-3' (SEQ ID NO : 29). Une première réaction de PCR a été réalisée avec la même amorce (SEQ ID NO : 29) et l'amorce directe de séquence SEQ ID NO : 22. Une réaction de PCR nichée a ensuite été effectuée avec l'amorce 5'-ATGCTATTTCAATGCTAGCTGCTATTT-3' (SEQ ID NO : 30) et l'amorce 5'-ATCTTTAAAATCGTTAGCTCGCCAGT-3' (SEQ ID NO: 31). Les produits de la deuxième PCR ont été clonés et séquencés. Les résultats du séquençage ont confirmé que l'ARN tRzatp9-L-PKTLS présent dans les cellules transformées avait bien les extrémités attendues, avec en particulier le triplet CCA à l'extrémité 3' de la séquence PKTLS (Figure 5).

### 3) Invalidation de l'ARNm atp9 mitochondrial par l'ARN tRzatp9-L-PKTLS importé

L'ARN tRzatp9-L-PKTLS étant importé dans les mitochondries des cellules de tabac transformées, son activité de clivage de sa cible, c'est-à-dire la séquence codante éditée de l'ARNm *atp9* de tabac (SEQ ID NO : 32), dans les organelles *in vivo* a été analysée. Pour cela, le taux d'ARNm *atp9* a été déterminé par RT-PCR quantitative (qRT-PCR) dans les ARN de cellules de tabac induites et non induites à l'oestradiol, transformées ou non transformées. La transcription inverse a été effectuée avec l'amorce 5'-GCAAACGATGCAATAGCTTCGGT-3' (SEQ ID NO : 33) et la réaction de PCR avec la même amorce couplée à l'amorce directe 5'-TGCTACAATTGCTTCAGCGGGA-3' (SEQ ID NO : 34). L'ARNm nucléaire codant pour l'actine et l'ARNm mitochondrial codant pour la protéine ribosomique RPL2 ont servi de références. Ils ont été amplifiés par qRT-PCR avec les couples d'amorces 5'-CGAAGAATTGCATGAGGAAGGGC-3' (directe) (SEQ ID NO : 35) - 5'-GCCAGTGGCCGTACAACAGGT-3' (réverse) (SEQ ID NO: 36) et 5'-CTTGCCCGCTTCCAATTGATG-3' (directe) (SEQ ID NO: 37) - 5'-CTGCCAAGCCGATAGGCGAA-3' (réverse) (SEQ ID NO : 38).

Les résultats, représentés à la Figure 6, ont montré que le taux d'ARNm *atp9* dans les cellules induites à l'oestradiol et exprimant l'ARN tRzatp9-L-PKTLS était réduit de 80% (+/-9%) par rapport à des cellules induites à l'oestradiol et n'exprimant pas l'ARN tRzatp9-L-PKTLS, démontrant l'invalidation dirigée ("knockdown") d'un ARNm mitochondrial dans des cellules de plantes.

Des analyses complémentaires par extension d'amorce fluorescente (FLOE *(« Fluorescently labelled Oligonucleotide Extension »* ou FLOE) (LLOYD et al., 2005, J Microbiol Methods, 60, 291-298) ont été effectuées avec les ARN des cellules de tabac transformées avec le plasmide pER8-tRzatp9LPKTLScHDV et induites ou non induites à l'oestradiol, afin de déterminer si la réduction du taux d'ARNm *atp9* était bien due au clivage spécifique du site ciblé (SEQ ID NO : 32) par le *trans*-ribozyme tRzatp9.

Les réactions ont été faites avec l'amorce 5'-CTAACGGACTTAGAATACGAATAAGAT-3' (SEQ ID NO : 39) marquée en 5' avec le fluorophore ROX.

Les résultats sont représentés à la Figure 7. Ces essais ont mis en évidence deux produits communs aux échantillons de cellules induites et non induites, un ARN de 357 nucléotides correspondant probablement à un transcrit initial et un ARN de 244 nucléotides correspondant à l'ARN mature, mais aussi un ARN de 168 nucléotides spécifique des cellules induites et correspondant au produit de clivage 3' de l'ARNm *atp9* par le *trans*-ribozyme tRzatp9 importé dans les mitochondries.

Ces résultats montrent donc qu'un *trans*-ribozyme associé à la séquence PKTLS du TYMV est fonctionnel dans les mitochondries des cellules de plantes et clive spécifiquement sa cible.

### 4) Phénotype induit par l'invalidation de l'ARNm atp9 mitochondrial résultant du clivage par l'ARN tRzatp9-L-PKTLS importé

Des essais de synthèse protéique *in organello* ont été effectués avec des mitochondries extraites des cellules de tabac transformées avec le plasmide pER8-tRzatp9LPKTLScHDV et induites ou non induites à l'oestradiol. En accord avec l'invalidation de l'ARNm *atp9,* la traduction de la protéine ATP9 était réduite en moyenne de 30% dans les mitochondries des cellules induites par rapport à celles des cellules non induites (voir Figure 8). L'activité respiratoire des mitochondries isolées des cellules induites était réduite dans des proportions similaires, tant au stade IV qu'au stade III (voir Figure 9). Enfin, les cellules transformées avec le plasmide pER8-tRzatp9LPKTLScHDV et induites à l'oestradiol présentaient un retard de croissance (voir Figure 10).

### EXEMPLE II : UTILISATION DE LA SEQUENCE PKTLS DU TYMV POUR IMPORTER DANS LES MITOCHONDRIES DES CELLULES DE PLANTES UN TRANS-RIBOZYME A TETE DE MARTEAU DIRIGE CONTRE L'ARN MITOCHONDRIAL mnc1

Dans le génome mitochondrial *d'Arabidopsis thaliana* (numéros d'accession gi:26556996 ou gi:49256807 dans la base de données GENBANK), la séquence codant pour l'ARN *mnc1* (nucléotides 159350-159643 en antisens) se trouve dans une longue région intergénique entre le gène *mttB* (*orfX*) et l'exon 1 du gène *nad4,* en orientation antisens par rapport à ces gènes de protéines. La Figure 11 (SEQ ID NO : 40) montre les éléments de l'ARN recombinant (polyribonucléotide) permettant d'importer dans les mitochondries des cellules de plantes un *trans*-ribozyme à tête de marteau dirigé contre l'ARN mitochondrial *mnc1.* Le *trans*-ribozyme à tête de marteau dirigé contre l'ARN *mnc1* (tRzmnc1) est associé à la séquence PKTLS du TYMV par l'intermédiaire d'une séquence de liaison (L) spécifique élaborée par bioinformatique avec l'aide du logiciel MFOLD.

### I. Validation in vitro de l'activité trans-ribozyme de l'ARN tRzmnc1-L-PKTLS

Avant d'initier la démarche *in vivo,* la fonctionnalité du système *trans-*ribozyme/cible a été établie *in vitro,* comme précédemment (cf. Exemple I), pour vérifier l'activité du ribozyme et la conservation de cette activité après association avec la séquence PKTLS du TYMV.

### 1) Constructions de gènes

L'ARN-cible (SEQ ID NO: 41) et l'ARN tRzmnc1-L-PKTLS ont été synthétisés par transcription *in vitro* à partir d'un produit PCR. La séquence d'ARN mitochondrial *mnc1 d'A. thaliana* (SEQ ID NO : 41) utilisée comme cible du *trans*-ribozyme tRzmnc1, a été amplifiée par PCR à partir d'ADN d'*A. thaliana* avec l'amorce directe 5'-AGCAAGAATTCGAATTGTAATACGACTCACTATAGGTGCTTTGCTCGCTCCGAC-3' (SEQ ID NO : 42) portant le promoteur de l'ARN polymérase du phage T7 (souligné) et l'amorce réverse 5'-GTACAGAATTCGTGACGTCTCTTGCTGGG-3' (SEQ ID NO : 43). La matrice ADN pour la synthèse de l'ARN tRzmnc1-L-PKTLS (SEQ ID NO : 40) a été assemblée par deux réactions de PCR consécutives, en partant du plasmide pCK-SPTYPKTLScHDV (décrit ci-dessus) renfermant la séquence PKTLS du TYMV, la première avec les amorces 5'-GAGCTTTTGCGAAACTGGCCACTTGCCTATAGCACAATCTATTCATGAACAATAG AAGATACACTTCCACCTAAGTTCTCG-3' (SEQ ID NO : 44) et de séquence SEQ ID NO: 10, la deuxième avec les amorces 5'-AGCAAGAATTCGAATTGTAATACGACTCACTATAGGAATCCAGCCTGATGAGCTT TTGCGAAACTGGCC-3' (SEQ ID NO : 45) (promoteur de l'ARN polymérase du phage T7) et de séquence SEQ ID NO : 10.

### 2) Tests de clivage in vitro

Afin de pouvoir visualiser son clivage, l'ARN-cible *mnc1* a été synthétisé *in vitro* sous une forme radioactive. Il a ensuite été incubé avec l'ARN tRzmnc1-L-PKTLS synthétisé à partir de la matrice PCR ou avec un ARN synthétique (commandé chez Sigma-Aldrich) correspondant strictement à la séquence du *trans*-ribozyme tRzmnc1 (5'-AAUCCAGCCUGAUGAGCUUUUGCGAAACUGGCC-3' ; SEQ ID NO : 46). La réaction a été testée en présence de concentrations de MgCl₂ susceptibles d'être retrouvées *in vivo* (IGAMBERDIEV and KLECZKOWSKI, 2001, Biochem J., 360, 225-231).

Les tests *in vitro* ont montré que le *trans*-ribozyme tRzmnc1 tel qu'il a été conçu était capable de cliver sa cible dans des conditions de MgCl₂ réduites (Figure 12A) et que son activité était conservée dans les mêmes conditions une fois qu'il était associé à la séquence PKTLS du TYMV par l'intermédiaire de la séquence de liaison spécifique L dans l'ARN tRzmnc1-L-PKTLS (Figure 12B).

### II. Clivage de l'ARN mnc1 dans les mitochondries des cellules de plantes in vivo

### 1) Construction de gènes

La séquence complète d'ADN (SEQ ID NO : 47) contenant les séquences exprimant le *trans*-ribozyme tRzmnc1, la séquence de liaison L, la séquence PKTLS du TYMV et le *cis*-ribozyme cHDV a été assemblée par amplification PCR à partir du plasmide pCK-SPTYPKTLScHDV (voir Exemple I) avec l'amorce directe 5'-GTACAAAGCTTAATCCAGCCTGATGAGCTTTTGCGAAACTGGCCACTTGCCTATA GCACAATCTATTCATGAACAATAGAAGATACACTTCCACCTAAGTTCTCG-3' (SEQ ID NO : 48) (site *HindIII* souligné) et l'amorce réverse de séquence SEQ ID NO : 14. Le fragment obtenu a été introduit dans le plasmide pUCAP (VAN ENGELEN et al., 1995, Transgenic Res. 4, 288-290) entre les sites *HindIIII* et *EcoRI,* puis ré-excisé avec les enzymes *AscI* et *PacI* et finalement cloné entre les sites *AscI* et *PacI* du plasmide pER8 (ZUO et al., 2000, Plant J., 24, 265-273), générant le plasmide pER8-tRzmnc1LPKTLScHDV. La construction a été ainsi insérée dans le plasmide pER8 dans la cassette d'expression sous contrôle du promoteur XVE inductible à l'oestradiol.

### 2) Transformation génétique des plantes

Le plasmide final pER8-tRzmnc1LPKTLScHDV a été utilisé pour transformer des inflorescences d'*A. thaliana* par l'intermédiaire d'*A. tumefaciens* ("floral dip", CLOUGH and BENT, 1998, Plant J., 16, 735-743). Des graines ont été récoltées pour le génotypage.

### EXEMPLE III : UTILISATION DE LA SEQUENCE PKTLS DU TYMV POUR IMPORTER DANS LES MITOCHONDRIES DES CELLULES DE PLANTES UN TRANS-RIBOZYME A TETE DE MARTEAU DIRIGE CONTRE L'ARN MITOCHONDRIAL or78

Un transcrit de 4,3 kb (numéro d'accession gi:14582613 dans la base de données GENBANK) de fonction inconnue et portant une phase de lecture ouverte non traduite (*orf78*) a été caractérisé antérieurement dans les mitochondries de pomme de terre *(Solanum tuberosum*) (SIQUEIRA et al., 2001, Biochim Biophys Acta, 1520, 203-211). Une partie de ce transcrit située hors de l'ORF *(Open Reading Frame)* est conservée dans des régions intergéniques des génomes mitochondriaux d'autres espèces comme *A. thaliana* ou *Brassica campestris* en orientation antisens par rapport à des gènes connus.

Une stratégie *trans*-ribozyme a été développée contre le transcrit de *S*. *tuberosum* (*or78*). La Figure 13 (SEQ ID NO : 49) montre les éléments du polyribonucléotide (ARN recombinant) élaboré pour cette stratégie. Le *trans*-ribozyme à tête de marteau dirigé contre l'ARN *or78* (tRzor78) est associé à la séquence PKTLS du TYMV par l'intermédiaire d'une séquence de liaison (L) spécifique élaborée par bioinformatique avec l'aide du logiciel MFOLD.

### I. Validation in vitro de l'activité trans-ribozyme de l'ARN tRzor78-L-PKTLS

La fonctionnalité du système *trans*-ribozyme/cible a été établie *in vitro,* comme précédemment (cf. Exemple I), pour vérifier l'activité du ribozyme et la conservation de cette activité après association avec la séquence PKTLS du TYMV.

### 1) Constructions de gènes

L'ARN-cible (SEQ ID NO : 50) et l'ARN tRzor78-L-PKTLS (SEQ ID NO : 49) ont été synthétisés par transcription *in vitro* à partir d'un produit PCR. La séquence d'ARN mitochondrial *or 78 de Solanum tuberosum* (correspondant aux nucléotides 987-1490, orientation sens dans la séquence identifiée sous le numéro d'accession gi:14582613 dans la base de données GENBANK ; SEQ ID NO : 50) utilisée comme cible du *trans*-ribozyme tRzorz78 a été amplifiée par PCR à partir d'ADN mitochondrial de S. *tuberosum* avec l'amorce directe 5'-AGCAAGAATTCGAATTGTAATACGACTCACTATAGGCCGATCTCAAGCTGGATG-3' (SEQ ID NO : 51) portant le promoteur de l'ARN polymérase du phage T7 (souligné) et l'amorce réverse 5'-GTACAGAATTCTTATCAACTCATAATAAGTAAGGC-3' (SEQ ID NO : 52). La matrice ADN pour la synthèse de l'ARN tRzor78-L-PKTLS (SEQ ID NO : 49) a été assemblée par deux réactions de PCR consécutives, en partant du plasmide pCK-SPTYPKTLScHDV (décrit ci-dessus) renfermant la séquence PKTLS du TYMV, la première avec les amorces 5'-ATGAGCTTTTGCGAAACGTTGTGACCAGCAGCTGCCAGTAACACACCTACGTGCG CTGCCAAACACTTCCACCTAAGTTCTCG-3' (SEQ ID NO : 53) et de séquence SEQ ID NO: 10, la deuxième avec les amorces 5'-AGCAAGAATTCGAATTGTAATACGACTCACTATAGGCCTATGTCCTGATGAGCTT TTGCGAAACGTTGT-3' (SEQ ID NO : 54) (promoteur de l'ARN polymérase du phage T7 souligné) et de séquence SEQ ID NO : 10.

### 2) Tests de clivage in vitro

Afin de pouvoir visualiser son clivage, l'ARN-cible *or78* a été synthétisé *in vitro* sous forme radioactive. Il a ensuite été incubé avec l'ARN tRzor78-L-PKTLS synthétisé à partir de la matrice PCR ou avec un ARN synthétique (commandé chez Sigma-Aldrich) correspondant strictement à la séquence du *trans*-ribozyme tRzor78 (5'-CCUAUGUCCUGAUGAGCUUUUGCGAAACGUUGU-3') (SEQ ID NO : 55). La réaction a été testée en présence de concentrations de MgCl₂ susceptibles d'être *retrouvées in vivo* (IGAMBERDIEV and KLECZKOWSKI, 2001, cité ci-dessus).

Les tests *in vitro* ont montré que le *trans*-ribozyme tRzor78 était capable de cliver sa cible dans des conditions de MgCl₂ réduites (Figure 14A) et que son activité était conservée dans les mêmes conditions une fois qu'il était associé à la séquence PKTLS du TYMV par l'intermédiaire de la séquence de liaison spécifique L dans l'ARN tRzor78-L-PKTLS (Figure 14B).

### II. Clivage de l'ARN or78 dans les mitochondries des cellules de plantes in vivo

### 1) Construction de gènes

La séquence d'ADN (SEQ ID NO: 56) exprimant le *trans*-ribozyme tRzor78, la séquence de liaison L, la séquence PKTLS du TYMV et le *cis*-ribozyme cHDV est assemblée par amplification PCR à partir du plasmide pCK-SPTYPKTLScHDV (voir Exemple I) avec l'amorce directe 5'-GTACAAAGCTTCCTATGTCCTGATGAGCTTTTGCGAAACGTTGTGACCAGCAGCT GCCAGTAACACACCTACGTGCGCTGCCAAACACTTCCACCTAAGTTCTCG-3' (SEQ ID NO : 57) (site *HindIII* souligné) et l'amorce réverse de séquence SEQ ID NO : 14. Le fragment obtenu est introduit dans le plasmide pUCAP (VAN *ENGELEN et al.,* 1995, cité ci-dessus) entre les sites *HindIIII* et *EcoRI,* puis ré-excisé avec les enzymes *AscI* et *PacI* et finalement cloné entre les sites *AscI* et *PacI* du plasmide pER8 (ZUO *et al.,* 2000, cité ci-dessus), générant le plasmide pER8-tRzor78LPKTLScHDV. La construction est ainsi insérée dans le plasmide pER8 dans la cassette d'expression sous contrôle du promoteur XVE inductible à l'oestradiol.

### 2) Transformation génétique des plantes

Le plasmide final pER8-tRzor78LPKTLScHDV est utilisé pour transformer des sections internodales de *S. tuberosum* par l'intermédiaire d'*A. tumefaciens* (MILLAM, 2006, Methods Mol. Biol., 344, 25-36.).

### EXEMPLE IV : COMPARAISON IN VITRO DE L'EFFICACITE DE CLIVAGE ENTRE 2 TYPES DE TRANS-RIBOZYMES A TETE DE MARTEAU DIRIGES CONTRE L'ARN MITOCHONDRIAL or78.

### 1) MATERIELS ET METHODES

4 constructions d'ARN différentes constituées d'un *trans*-ribozyme à tête de marteau dirigé contre l'ARN mitochondrial *or78* (SEQ ID NO: 50), d'une séquence de liaison (L) et de la séquence PKTLS du TYMV (PKTLS) (voir Figure 15) ont été testées quant à leur efficacité de clivage de l'ARN mitochondrial *or78* (ARN-cible) :
- tRzor78-L-PKTLS (SEQ ID NO : 49) : le *trans*-ribozyme (tRzor78) de cette construction présente au niveau de sa tige-boucle II, une boucle constituée de 4 nucléotides (UUUU) et une hélice II constituée de 2 paires de nucléotides (G-C et C-G), et ne contient pas de structure de stabilisation tertiaire ; 8 nucléotides contigus s'hybrident à l'ARN-cible en 5' du *trans*-ribozyme (hélice I), et 7 nucléotides contigus s'hybrident à l'ARN-cible en 3' (hélice III) du *trans*-ribozyme ;
- tRzor78TS-L-PKTLS (SEQ ID NO : 58) : le *trans*-ribozyme (tRzor78TS) de cette construction est stabilisé par des interactions tertiaires (« *tertiary stabilized* ») grâce à l'addition d'une tige-boucle supplémentaire (par rapport au *trans*-ribozyme tRzor78) en position 5' qui établit des interactions tertiaires avec la tige-boucle II. Il présente au niveau de sa tige-boucle II, une boucle constituée de 4 nucléotides (GAAA) et une hélice II constituée de 4 paires de nucléotides (G-C, U-A, C-G, G-C) ; 6 nucléotides contigus s'hybrident à l'ARN-cible en 5' (hélice I) et en 3' (hélice III) du *trans*-ribozyme ;
- tRzor78/6+6-L-PKTLS (SEQ ID NO: 59): le *trans*-ribozyme (tRzor78/6+6; aux positions 2 à 32) de cette construction présente au niveau de sa tige-boucle II, une boucle constituée de 4 nucléotides (UUUU) et une hélice II constituée de 2 paires de nucléotides (G-C et C-G), et ne contient pas de structure de stabilisation tertiaire ; 6 nucléotides contigus s'hybrident à l'ARN-cible en 5' (hélice I) et en 3' (hélice III) du *trans*-ribozyme ;
- tRzor78TS/8+7-L-PKTLS (SEQ ID NO : 60): le *trans*-ribozyme (tRzor78TS/8+7) de cette construction est stabilisé par des interactions tertiaires (« *tertiary stabilized* ») grâce à l'addition d'une tige-boucle supplémentaire (par rapport au *trans-*ribozyme tRzor78) en position 5' qui établit des interactions tertiaires avec la tige-boucle II. Il présente au niveau de sa tige-boucle II, une boucle constituée de 4 nucléotides (GAAA) et une hélice II constituée de 4 paires de nucléotides (G-C, U-A, C-G, G-C) ; 8 nucléotides contigus s'hybrident à l'ARN-cible en 5' du *trans*-ribozyme (hélice I), et 7 nucléotides contigus s'hybrident à l'ARN-cible en 3' du *trans*-ribozyme (hélice III).

La synthèse de l'ARN-cible *or78* (SEQ ID NO : 50) et de l'ARN tRzor78-L-PKTLS (SEQ ID NO : 49) par transcription *in vitro* à partir d'un produit PCR a été effectuée comme décrit précédemment (voir Exemple III.I.1).

La même stratégie a été développée pour produire les trois autres ARN recombinants ribozyme-L-PKTLS. Les matrices ADN pour la synthèse des ARN tRzor78TS-L-PKTLS, tRzor78/6+6-L-PKTLS et tRzor78TS/8+7-L-PKTLS ont été assemblées par deux réactions de PCR consécutives, en partant du plasmide pCK-SPTYPKTLScHDV (décrit ci-dessus) contenant la séquence PKTLS du TYMV (le promoteur de l'ARN polymérase du phage T7 est souligné) :
- pour la synthèse de tRzor78TS-L-PKTLS, la première PCR a été effectuée avec l'amorce directe 5'-GTCGGAAACGACGAAACGTTGACTTGCCTATAGCACAATCTATTCATGAACAATA GAAGATACACTTCCACCTAAGTTCTCG-3' (SEQ ID NO : 61) et la deuxième PCR a été effectuée avec l'amorce directe 5'AGCAAGAATTCGAATTGTAATACGACTCACTATAGGCTAAGGCAAACGCTATG CTATGTCCTGATGAGTCGGAAACGACGAAACGTTG-3' (SEQ ID NO : 62) ;
- pour la synthèse de tRzor78/6+6-L-PKTLS, la première PCR a été effectuée avec l'amorce directe 5'-GATGAGCTTTTGCGAAACGTTGGAGAAGAAGCTGCCAGTAACACACCTACGTGC GCTGCCAAACACTTCCACCTAAGTTCTCG-3' (SEQ ID NO : 63) et la deuxième PCR a été effectuée avec l'amorce directe 5'AGCAAGAATTCGAATTGTAATACGACTCACTATAGGTATGTCCTGATGAGCTTT TGCGAAACGTTG-3' (SEQ ID NO : 64) ;
- pour la synthèse de tRzor78TS/8+7-L-PKTLS, la première PCR a été effectuée avec l'amorce directe 5'-TCGGAAACGACGAAACGTTGTACTTGAATATAGCACAATCTATTCATGAACAAAA GAAGATACACTTCCACCTAAGTTCTCG-3' (SEQ ID NO : 65) et la deuxième PCR a été effectuée avec l'amorce directe 5'-AGCAAGAATTCGAATTGTAATACGACTCACTATAGGCTAAGGCAAACGCTATGCC CTATGTCCTGATGAGTCGGAAACGACGAAACGTTGT-3' (SEQ ID NO : 66).

L'amorce 5'-TGGTTCCGATGACCCTCGG-3' (SEQ ID NO: 10), complémentaire de l'extrémité 3' de la PKTLS, a servi d'amorce réverse pour toutes les réactions PCR.

### 2) Tests de clivage in vitro

L'activité de clivage des différents *trans*-ribozymes à tête de marteau combinés à une séquence de liaison (L) et à la séquence PKTLS de TYMV a été testée dans un milieu réactionnel contenant 15 nM d'ARN-cible *or78* non radioactif, 50 fmol (30 000 cpm) d'ARN-cible *or78* marqué au [³²P], 150 nM d'ARN ribozyme-L-PKTLS et 50 mM de tampon Tris-HCl à pH 7,5. Le milieu réactionnel a d'abord été porté pendant 2 min à 75°C et refroidi très lentement (0,3°C par min) jusqu'à 25°C, afin de dénaturer les structures qui se forment dans l'ARN-cible et l'ARN catalytique durant la transcription par l'ARN polymérase T7 et de favoriser la formation du complexe spécifique *trans*-ribozyme / ARN-cible. La réaction de clivage proprement dite a été réalisée par incubation du milieu réactionnel pendant 1 à 5 heures à 25°C, en présence de différentes concentrations de MgCl₂. Les produits de clivage ont ensuite été séparés par électrophorèse sur gel de polyacrylamide à 8% ou 10% (p/v) / urée 8 M dans du tampon Tris-borate 90 mM contenant 2 mM d'EDTA (1x TBE). Les gels ont été fixés, séchés et analysés par autoradiographie.

### 2) RESULTATS

L'ARN-cible *or78* a été synthétisé *in vitro* sous forme radioactive afin de pouvoir visualiser son clivage. Il a ensuite été incubé avec les ARN recombinants tRzor78-L-PKTLS (SEQ ID NO : 49), tRzor78TS-L-PKTLS (SEQ ID NO : 58), tRzor78/6+6-L-PKTLS (SEQ ID NO: 59) et tRzor78TS/8+7-L-PKTLS (SEQ ID NO: 60) synthétisés par *transcription in vitro* à partir des matrices PCR correspondantes. La réaction a été testée en présence de concentrations de MgCl₂ susceptibles d'être retrouvées *in vivo* (1mM).

Les résultats sont représentés à la Figure 16. Ces résultats montrent que l'ensemble des ARN recombinants testés clive l'ARN-cible *or78* aussi bien en présence de 1 mM MgCl₂ qu'en l'absence de Mg²⁺, ce qui est particulièrement favorable pour une utilisation *in vivo.*

Toutefois, les tests comparatifs *in vitro* ont montré que l'ARN recombinant tRzor78-L-PKTLS avait une meilleure efficacité de clivage par rapport à celle des trois autres ARN recombinants. Cette meilleure efficacité a été retrouvée aussi bien en présence de 1 mM MgCl₂ qu'en l'absence de Mg²⁺.

## Revendications

1. Polyribonucléotide comprenant, de son extrémité 5' vers son extrémité 3', *trans*-ribozyme à tête de marteau dirigé contre un ARN mitochondrial de plante et une structure en forme d'ARNt aminoacylable par la valine.

2. Polyribonucléotide selon la revendication 1, **caractérisé en ce que** ledit *trans*-ribozyme à tête de marteau est constitué de 24 à 100 nucléotides, de préférence de 30 à 40 nucléotides.

3. Polyribonucléotide selon la revendication 1 ou la revendication 2, **caractérisé en ce que** ledit *trans*-ribozyme à tête de marteau présente au niveau de sa tige-boucle II, une boucle constituée de 4 nucléotides, et une hélice II constituée de 2 paires de nucléotides.

4. Polyribonucléotide selon l'une quelconque des revendications 1 à 3, **caractérisé en ce que** ledit *trans*-ribozyme à tête de marteau s'hybride audit ARN mitochondrial par l'intermédiaire de 3 à 50 nucléotides contigus, en position 5' dudit *trans-*ribozyme et par l'intermédiaire de 3 à 50 nucléotides contigus, en position 3' dudit *trans-*ribozyme.

5. Polyribonucléotide selon l'une quelconque des revendications 1 à 4, **caractérisé en ce que** ledit *trans*-ribozyme et ladite structure en forme d'ARNt aminoacylable par la valine sont séparés par une séquence ARN de liaison.

6. Polyribonucléotide selon l'une quelconque des revendications 1 à 5, **caractérisé en ce que** ledit *trans*-ribozyme ou ladite séquence ARN de liaison et ladite structure en forme d'ARNt aminoacylable par la valine sont séparés par une structure en pseudo-noeud.

7. Polyribonucléotide selon l'une quelconque des revendications 1 à 6, **caractérisé en ce que** le dernier nucléotide en position 3' de ladite structure en forme d'ARNt aminoacylable par la valine est une adénine.

8. Polyribonucléotide selon l'une quelconque des revendications 1 à 7, **caractérisé en ce que** ladite structure en forme d'ARNt aminoacylable par la valine, contenant optionnellement une adénine en position 3', est associée en position 3' à un ribozyme autoclivable en *cis* en position 5' (*cis*-ribozyme).

9. Polyribonucléotide selon l'une quelconque des revendications 1 à 8, **caractérisé en ce que** l'ARN mitochondrial de plante est choisi parmi l'ARN messager du gène *atp9,* l'ARN *mncl* et l'ARN *or78.*

10. Polyribonucléotide selon l'une quelconque des revendications 1 à 9, **caractérisé en ce que** ladite structure en forme d'ARNt aminoacylable par la valine et/ou ladite structure en pseudo-noeud sont celles contenues dans le génome d'un virus, de préférence d'un Tymovirus, de préférence encore du *Turnip yellow mosaic virus* (TYMV).

11. Polyribonucléotide selon l'une quelconque des revendications 8 à 10, **caractérisé en ce que** ledit *cis*-ribozyme est issu du génome d'un virus, de préférence d'un *Hepatitis Delta virus* (HDV).

12. Polyribonucléotide selon la revendication 11, **caractérisé en ce qu'**il est choisi parmi les séquences SEQ ID NO : 2,40, 49, 58, 59 et 60.

13. Polydésoxyribonucléotide exprimant un polyribonucléotide selon l'une quelconque des revendications 1 à 12.

14. Polydésoxyribonucléotide selon la revendication 13, **caractérisé en ce qu'**il est choisi parmi les séquences SEQ ID NO : 1, 47 et 56.

15. Utilisation d'une structure en forme d'ARNt aminoacylable par la valine telle que définie à l'une quelconque des revendications 1 et 10, pour importer dans les mitochondries des cellules de plantes un *trans*-ribozyme à tête de marteau dirigé contre un ARN mitochondrial de plante.

16. Cassette d'expression recombinante, **caractérisée en ce qu'**elle comprend un polydésoxyribonucléotide selon la revendication 13 ou la revendication 14 sous le contrôle d'un promoteur transcriptionnel approprié, de préférence un promoteur fonctionnel dans les cellules de plantes.

17. Cassette d'expression selon la revendication 16, **caractérisée en ce que** ledit promoteur est un promoteur inductible ou un promoteur spécifique du pollen et/ou des anthères.

18. Vecteur recombinant, **caractérisé en ce qu'**il comprend un polydésoxyribonucléotide selon la revendication 13 ou la revendication 14 ou une cassette d'expression selon l'une quelconque des revendications 16 et 17.

19. Cellule hôte de préférence une cellule de plante, **caractérisée en ce qu'**elle comprend une cassette d'expression selon l'une quelconque des revendications 16 et 17 ou un vecteur recombinant selon la revendication 18.

20. Plante génétiquement transformée par au moins un polydésoxyribonucléotide selon la revendication 13 ou la revendication 14 ou une cassette d'expression selon l'une quelconque des revendications 16 et 17.

21. Procédé d'obtention d'une plante transgénique possédant une stérilité mâle cytoplasmique, **caractérisé en ce qu'**il comprend les étapes suivantes :
a) obtenir une cellule de plante comprenant une cassette d'expression telle que définie à la revendication 16 ou 17 qui comprend une séquence exprimant un ribozyme à tête de marteau dirigé contre l'ARNm *atp9,* l'ARN *mnc1* ou l'ARN *or78,* et
b) régénérer à partir de la cellule de plante obtenue à l'étape a) une plante transgénique exprimant ledit ribozyme à tête de marteau dirigé contre l'ARNm *atp9*, l'ARN *mnc1* ou l'ARN *or78.*

22. Plante transgénique susceptible d'être obtenue par le procédé selon la revendication 21, et comprenant le polyribonucleotide tel que défini à l'une quelconque des revendications 1 à 10.

## Patentansprüche

1. Polyribonukleotid, enthaltend von seinem 5'-Ende zu seinem 3'-Ende ein Hammerkopf-*trans*-Ribozym, gegen eine pflanzliche mitochondriale RNS und eine Struktur in Form einer durch Valin aminoacylierbaren tRNS gerichtet.

2. Polyribonukleotid nach Anspruch 1, **dadurch gekennzeichnet, dass** das Hammerkopf-*trans*-Ribozym aus 24 bis 100 Nukleotiden, vorzugsweise 30 bis 40 Nukleotiden, aufgebaut ist.

3. Polyribonukleotid nach Anspruch 1 oder Anspruch 2, **dadurch gekennzeichnet, dass** das Hammerkopf-*trans*-Ribozym in Höhe seiner Stamm II-Schleife eine Schleife, welche aus 4 Nukleotiden aufgebaut ist, und eine Helix II, welche aus 2 Nukleotidpaaren aufgebaut ist, aufweist.

4. Polyribonukleotid nach irgendeinem der Ansprüche 1 bis 3, **dadurch gekennzeichnet, dass** das Hammerkopf-*trans*-Ribozym an die mitochondriale RNS über angrenzende 3 bis 50 Nukleotide in der 5'-Position des *trans-*Ribozyms und über angrenzende 3 bis 50 Nukleotide in der 3'-Position des *trans*-Ribozyms hybridisiert.

5. Polyribonukleotid nach irgendeinem der Ansprüche 1 bis 4, **dadurch gekennzeichnet, dass** das *trans*-Ribozym und die Struktur in Form einer durch Valin aminoacylierbaren tRNS über eine RNS-Verknüpfungssequenz getrennt sind.

6. Polyribonukleotid nach irgendeinem der Ansprüche 1 bis 5, **dadurch gekennzeichnet, dass** das *trans*-Ribozym oder die RNS-Verknüpfungssequenz und die Struktur in Form der durch Valin aminoacylierbaren tRNS getrennt sind durch eine Pseudohaarnadelstruktur.

7. Polyribonukleotid nach irgendeinem der Ansprüche 1 bis 6, **dadurch gekennzeichnet, dass** das letzte Nukleotid in 3'-Position der Struktur in Form einer durch Valin aminoacylierbaren tRNS ein Adenin ist.

8. Polyribonukleotid nach irgendeinem der Ansprüche 1 bis 7, **dadurch gekennzeichnet, dass** die Struktur in Form der durch Valin aminoacylierbaren tRNS, welche gegebenenfalls ein Adenin in der 3'-Position enthält, an der 3'-Position an ein Ribozym gebunden ist, welches an *cis* in 5'-Position spaltbar ist (*cis*-Ribozym).

9. Polyribonukleotid nach irgendeinem der Ansprüche 1 bis 8, **dadurch gekennzeichnet, dass** die pflanzliche mitochondriale RNS ausgewählt wird aus Messenger-RNS des Gens *atp9, mncl* RNS und *or78* RNS.

10. Polyribonukleotid nach irgendeinem der Ansprüche 1 bis 9, **dadurch gekennzeichnet, dass** die Struktur in Form einer durch Valin aminoacylierbaren tRNS und/oder die Pseudohaarnadelstruktur im Genom eines Virus enthalten sind, vorzugsweise eines Tymovirus, besonders bevorzugt *Turnip yellow mosaic virus* (TYMV).

11. Polyribonukleotid nach irgendeinem der Ansprüche 8 bis 10, **dadurch gekennzeichnet, dass** das *cis*-Ribozym aus einem Virusgenom stammt, vorzugsweise aus dem *Hepatitis Delta Virus* (HDV).

12. Polyribonukleotid nach Anspruch 11, **dadurch gekennzeichnet, dass** es ausgewählt ist aus den Sequenzen mit den SEQ ID NO: 2, 40, 49, 58, 59 und 60.

13. Polydesoxyribonukleotid, ein Polyribonukleotid gemäß einem der Ansprüche 1 bis 12 exprimierend.

14. Polydesoxyribonukleotid nach Anspruch 13, **dadurch gekennzeichnet, dass** es ausgewählt ist aus den Sequenzen mit den SEQ ID NO: 1, 47 und 56.

15. Verwendung einer Struktur in Form einer durch Valin aminoacylierbaren tRNS, welche gemäß einem der Ansprüche 1 bis 10 definiert ist, zum Einführen eines Hammerkopf-*trans*-Ribozyms, welches gegen eine pflanzliche mitochondriale RNS gerichtet ist, in die Mitochondrien von Pflanzenzellen.

16. Rekombinante Expressionskassette, **dadurch gekennzeichnet, dass** sie ein Polydesoxyribonukleotid gemäß Anspruch 13 oder gemäß Anspruch 14 unter der Kontrolle eines geeigneten Transkriptionspromotors umfasst, insbesondere eines funktionellen Promotors in den Pflanzenzellen.

17. Expressionskassette nach Anspruch 16, **dadurch gekennzeichnet, dass** der Promotor ein induzierbarer Promotor oder ein spezifischer Pollen- und/oder Antherenpromotor ist.

18. Rekombinanter Vektor, **dadurch gekennzeichnet, dass** er ein Polydesoxyribonukleotid gemäß Anspruch 13 oder Anspruch 14 oder eine Expressionskassette gemäß einem der Ansprüche 16 und 17 umfasst.

19. Vorzugsweise pflanzliche Wirtszelle, **dadurch gekennzeichnet, dass** sie eine Expressionskassette gemäß einem der Ansprüche 16 und 17 oder einen rekombinanten Vektor gemäß Anspruch 18 umfasst.

20. Transgene Pflanze, transformiert mit wenigstens einem Polydesoxyribonukleotid gemäß Anspruch 13 oder Anspruch 14 oder einer Expressionskassette gemäß irgendeinem der Ansprüche 16 und 17.

21. Verfahren zum Erhalten einer transgenen Pflanze, welche eine zytoplasmatische männliche Sterilität aufweist, **dadurch gekennzeichnet, dass** es die folgenden Schritte umfasst:
a) Erhalten einer Pflanzenzelle, welche eine Expressionskassette, wie sie in Anspruch 16 oder 17 definiert ist, umfasst, welche eine Expressionssequenz für ein Hammerkopf-Ribozym umfasst, das gegen die *atp9* mRNS, *mncl* RNS oder *or78* RNS gerichtet ist, und
b) Regenerieren aus der in Schritt a) erhaltenen Pflanzenzelle einer transgenen Pflanze, welche das Hammerkopf-Ribozym exprimiert, welches gegen *atp9* mRNS, *mncl* RNS oder *or78* RNS gerichtet ist.

22. Transgene Pflanze, welche geeignet ist, gemäß dem Verfahren nach Anspruch 21 erhalten zu werden, und welche die Polyribonukleotide, wie in irgendeinem der Ansprüche 1 bis 10 definiert, umfasst.

## Claims

1. A polyribonucleotide comprising, from its 5' end to its 3' end, a *trans* hammerhead ribozyme directed against a plant mitochondrial RNA and a tRNA-like structure that can be aminoacylated by valine.

2. The polyribonucleotide as claimed in claim 1, **characterized in that** said *trans* hammerhead ribozyme consists of from 24 to 100 nucleotides, preferably from 30 to 40 nucleotides.

3. The polyribonucleotide as claimed in claim 1 or claim 2, **characterized in that** said *trans* hammerhead ribozyme has, in its stem-loop II, a loop consisting of 4 nucleotides, and a helix II consisting of 2 nucleotide pairs.

4. The polyribonucleotide as claimed in any one of claims 1 to 3, **characterized in that** said *trans* hammerhead ribozyme hybridizes to said mitochondrial RNA by means of from 3 to 50 contiguous nucleotides in the 5' position of said *trans*-ribozyme and by means of from 3 to 50 contiguous nucleotides in the 3' position of said trans-ribozyme.

5. The polyribonucleotide as claimed in any one of claims 1 to 4, **characterized in that** said *trans-*ribozyme and said tRNA-like structure that can be aminoacylated by valine are separated by a linker RNA sequence.

6. The polyribonucleotide as claimed in any one of claims 1 to 5, **characterized in that** said *trans-*ribozyme or said linker RNA sequence and said tRNA-like structure that can be aminoacylated by valine are separated by an upstream pseudoknot.

7. The polyribonucleotide as claimed in any one of claims 1 to 6, **characterized in that** the last nucleotide in the 3' position of said tRNA-like structure that can be aminoacylated by valine is an adenine.

8. The polyribonucleotide as claimed in any one of claims 1 to 7, **characterized in that** said tRNA-like structure that can be aminoacylated by valine, optionally containing an adenine in the 3' position, is combined, in the 3' position, with a ribozyme that is self-cleaving in *cis* in the 5' position (cis-ribozyme).

9. The polyribonucleotide as claimed in any one of claims 1 to 8, **characterized in that** the plant mitochondrial RNA is chosen from the messenger RNA of the *atp9* gene, the *mncl* RNA and the *or78* RNA.

10. The polyribonucleotide as claimed in any one of claims 1 to 9, **characterized in that** said tRNA-like structure that can be aminoacylated by valine and/or said upstream pseudoknot are those contained in the genome of a virus, preferably a Tymovirus, more preferably a *Turnip yellow mosaic virus* (TYMV).

11. The polyribonucleotide as claimed in any one of claims 8 to 10, **characterized in that** said cis-ribozyme is derived from the genome of a virus, preferably a *Hepatitis Delta virus* (HDV).

12. The polyribonucleotide as claimed in claim 11, **characterized in that** it is chosen from the sequences SEQ ID Nos. 2, 40, 49, 58, 59 and 60.

13. A polydeoxyribonucleotide expressing a polyribonucleotide as claimed in any one of claims 1 to 12.

14. The polydeoxyribonucleotide as claimed in claim 13, **characterized in that** it is chosen from the sequences SEQ ID Nos. 1, 47 and 56.

15. The use of a tRNA-like structure that can be aminoacylated by valine as defined in any one of claims 1 and 10, for importing into plant cell mitochondria a *trans* hammerhead ribozyme directed against a plant mitochondrial RNA.

16. A recombinant expression cassette, **characterized in that** it comprises a polydeoxyribonucleotide as claimed in claim 13 or claim 14 under the control of an appropriate transcription promoter, preferably a promoter that is functional in plant cells.

17. The expression cassette as claimed in claim 16, **characterized in that** said promoter is an inducible promoter or a pollen- and/or anther-specific promoter.

18. A recombinant vector, **characterized in that** it comprises a polydeoxyribonucleotide as claimed in claim 13 or claim 14 or an expression cassette as claimed in either one of claims 16 and 17.

19. A host cell, preferably a plant cell, **characterized in that** it comprises an expression cassette as claimed in either one of claims 16 and 17 or a recombinant vector as claimed in claim 18.

20. A plant genetically transformed with at least one polydeoxyribonucleotide as claimed in claim 13 or claim 14 or one expression cassette as claimed in either one of claims 16 and 17.

21. A method for obtaining a transgenic plant having cytoplasmic male sterility, **characterized in that** it comprises the following steps:
a) obtaining a plant cell comprising an expression cassette as defined in claim 16 or 17 which comprises a sequence expressing a hammerhead ribozyme directed against the *atp9* mRNA, the *mncl* RNA or the *or78* RNA, and
b) regenerating, from the plant cell obtained in step a), a transgenic plant expressing said hammerhead ribozyme directed against the *atp9* mRNA, the *mncl* RNA or the *or78* RNA.

22. A transgenic plant that can be obtained by means of the method as claimed in claim 21 and comprising the polyribonucleotide as defined in any one of claims 1 to 10.
